# EUROPEAN PATENT SPECIFICATION

(11) **EP 1 204 870 B1**
(45) Date of publication and mention of the grant of the patent: **20.01.2010**
(21) Application number: 00955336.3
(22) Date of filing: 02.08.2000
(51) Int. Cl.: G01N 33/72, A61P 7/00, A61K 35/18, A61K 38/42

(54) **METHOD FOR DETERMINING PHYSIOLOGICAL EFFECTS OF HEMOGLOBIN**
METHODE ZUR BESTIMMUNG DER PHYSIOLOGISCHEN WIRKUNGEN VON HÄMOGLOBIN
PROCEDE POUR DETERMINER LES EFFETS PHYSIOLOGIQUES DE L'HEMOGLOBINE

(30) Priority: 02.08.1999 US 146680 P
(43) Date of publication of application: 15.05.2002
(73) Proprietor: Duke University, Durham, NC 27710 (US); Research and Development Institute, Inc., Bozeman, MT 59715 (US)
(72) Inventor: STAMLER, Jonathan, S., Chapel Hill, NC 27514 (US); GOW, Andrew, J., Durham, NC 27713 (US); SINGEL, David, J., Bozeman, MT 59715 (US)
(74) Representative: Crump, Julian Richard John
(86) International application number: PCT/US2000/021101
(87) International publication number: WO 2001/009616

(56) References cited:
- WO-A-98/34955
- GOW ANDREW J ET AL: "The oxyhemoglobin reaction of nitric oxide." PROCEEDINGS OF THE NATIONAL ACADEMY OF SCIENCES OF THE UNITED STATES, vol. 96, no. 16, 3 August 1999 (1999-08-03), pages 9027-9032, XP002157932 Aug. 3, 1999 ISSN: 0027-8424 cited in the application
- YONETANI TAKASHI ET AL: "Electron paramagnetic resonance and oxygen binding studies of alpha-nitrosyl hemoglobin: A novel oxygen carrier having no-assisted allosteric functions." JOURNAL OF BIOLOGICAL CHEMISTRY, vol. 273, no. 32, 7 August 1998 (1998-08-07), pages 20323-20333, XP002157933 ISSN: 0021-9258
- ASCENZI PAOLO ET AL: "Cooperative effect of inositol hexakisphosphate, bezafibrate, and clofibric acid on the spectroscopic properties of the nitric oxide derivative of ferrous human hemoglobin." JOURNAL OF INORGANIC BIOCHEMISTRY, vol. 50, no. 4, 1993, pages 263-272, XP000980291 ISSN: 0162-0134
- GOW A J ET AL: "REACTIONS BETWEEN NITRIC OXIDE AND HAEMOGLOBIN UNDER PHYSIOLOGICAL CONDITIONS" NATURE,GB,MACMILLAN JOURNALS LTD. LONDON, vol. 391, 8 January 1998 (1998-01-08), pages 169-173, XP002067325 ISSN: 0028-0836 cited in the application
- MCMAHON TIMOTHY JOSEPH ET AL: "Functional coupling of oxygen binding and vasoactivity in S-nitrosohemoglobin." JOURNAL OF BIOLOGICAL CHEMISTRY, vol. 275, no. 22, 2 June 2000 (2000-06-02), pages 16738-16745, XP002157934 ISSN: 0021-9258 cited in the application
- STAMLER JONATHAN S ET AL: "Blood flow regulation by S-nitrosohemoglobin in the physiological oxygen gradient." SCIENCE (WASHINGTON D C), vol. 276, no. 5321, 1997, pages 2034-2037, XP002157935 ISSN: 0036-8075 cited in the application
- JIA L ET AL: "S-NITROSOHAEMOGLIBIN: A DYNAMIC ACTIVITY OF BLOOD INVOLVED IN VASCULAR CONTROL" NATURE,GB,MACMILLAN JOURNALS LTD. LONDON, vol. 391, 21 March 1996 (1996-03-21), pages 221-226, XP002067324 ISSN: 0028-0836 cited in the application
- CRAEGER ET AL.: "Effect of N-acetylcysteine on endothelium-dependent vasodilation in humans" CIRCULATION, vol. 86, no. 4, 1992, page I620, XP009102410

## Description

### BACKGROUND OF THE INVENTION

Nitric oxide has been associated with many physiological effects, among them, smooth muscle contraction, vasodilation, inflammation responses, and inhibition of platelet adhesion and aggregation. Finding the natural reservoirs of NO and finding ways to regulate the levels of biologically available NO and its alternative forms would provide the means to control these physiological effects.

The interaction of hemoglobin with nitric oxide (NO) can be manifested in a number of ways, for example:
1.) quenching (Equation 1, producing metHb, a vasoconstrictor);
2.) trapping NO, or loading of Hb (Equation 2, producing iron nitrosyl-hemoglobin, a vasoconstrictor);
3.) arming of Hb (producing SNO-oxyHb, a vasoconstrictor); and
4.) release (deoxyHb acting as a donor of NO, more specifically, a vasodilator).

The structural properties of a hemoglobin and its ionic environment have been observed to determine the physiological effects produced by administration of a hemoglobin composition to a human or other mammal. A method to predict such physiological effects would be desirable to allow the rational use of hemoglobin compositions in methods of therapy to provide a blood substitute or other therapeutic. A more thorough understanding of the pathways by which NO or its biological equivalent is transferred and transported would allow more methods by which the physiological effects of NO can be regulated.

### SUMMARY OF THE INVENTION

NO preferentially binds to the minor population of the hemoglobin's vacant hemes in a cooperative manner, nitrosylates hemoglobin thiols, or reacts with liberated superoxide in solution. The distribution of minor forms of NO-modified hemoglobin (herein, S-nitrosohemoglobin or iron nitrosylhemoglobin) can be tested in various hemoglobin compositions and the results can be used to predict whether a composition comprising hemoglobin will, for example, scavenge, load, eliminate, or donate NO. Methods of therapy can take advantage of the properties of hemoglobin in different buffers.

Hemoglobin of mammalian red blood cells (RBCs) is the largest reservoir of nitric oxide (NO) in the body (Jia, L., et al., Nature, 380:221-226, 1996). There is considerable evidence for the proposition that it is also a major locus of NO throughput, alternately functioning to conserve (Fe[II]NO), generate (Cysβ93NO) and release NO bioactivity in order to optimize oxygen delivery in the respiratory cycle (Jia, L., et al., Nature, 380:221-226, 1996; Stamler, J.S., et al., Science, 276:2034-2037, 1997; Gow, A.J., et al., Nature, 391:169-173, 1998; Gow, A.J., et al., Proc. Natl. Acad. Sci. USA, 96:9027-9032, 1999; McMahon, T.J., et al., J. Biol. Chem., 275:16738-16745, 2000). However, while both O₂ and NO diffuse into the RBC, only O₂ can diffuse out (Gow, A.J., et al., Nature, 391:169-173, 1998; Gow, A.J., et al., Proc. Natl. Acad. Sci. USA, 96:9027-9032, 1999; McMahon, T.J., et al., J. Biol. Chem., 275:16738-16745, 2000). Thus if RBCs are to dilate blood vessels, then not only must they transform NO into bioactive Cysβ93NO, but a previously undescribed mechanism must export this vasoactivity, and current models of NO-mediated intercellular communication must be revised. Herein it is described that, in human erythrocytes, hemoglobin-derived S-nitrosothiol (SNO)-generated from imported NO- is associated predominantly with the RBC membrane, and principally with cysteine residues in the hemoglobin-binding cytoplasmic domain of the anion exchanger AE1 (band 3 protein). Interaction with AE1 promotes the deoxygenated structure in SNO-Hb that drives NO group transfer to the membrane. Vasodilatory activity is released from this membrane precinct by deoxygenation to relax vascular smooth muscle. Thus, the oxygen-regulated cellular mechanism that couples synthesis and export of Hb-derived NO bioactivity is based at least in part upon formation of AE1-SNO at the RBC membrane-cytosol interface. These findings can be used to produce methods of therapy for medical disorders characterized by red blood cell membrane defects, and for a variety of hypercoaguable and vasculopathic states.

In one embodiment the invention is a method for determining the predominant physiological effect of a composition comprising hemoglobin, comprising the steps of obtaining EPR or UV spectra of iron-nitrosyl hemoglobin derivatives formed by incubation of limiting NO with hemoglobin at various degrees of oxygen saturation; determining from the results in a) whether the composition shows non-cooperativity or cooperativity in binding of NO to the hemoglobin; and, if the composition shows cooperativity, then assaying the composition at an oxygen saturation at which the hemoglobin is approximately 99% oxyhemoglobin and 1% deoxyhemoglobin, under limiting NO concentration, to determine whether S-nitrosohemogiobin or iron nitrosyl-hemoglobin is greater; wherein, if the composition shows non-cooperativity, then the predominant physiological effect of the composition is elimination of NO; if the composition shows cooperativity and if S-nitroso-hemoglobin is greater, then the predominant physiological effect of the composition is delivering NO; and if the composition shows cooperativity and if iron nitrosyl-hemoglobin is greater, then the predominant physiological effect of the composition is trapping of NO.

A further method for determining the predominant physiological effect of a composition comprising hemoglobin arises out of similar steps, wherein, if the composition shows non-cooperativity, then the predominant physiological effect of the composition is vasoconstriction; if the composition shows cooperativity and if the most prevalent species of NO-modified hemoglobin is *S*-nitrosohemoglobin, then the predominant physiological effect of the composition is vasodilation; and if the composition shows cooperativity and if iron nitrosyl-hemoglobin is greater, then the predominant physiological effect of the composition is vasoconstriction.

In a method of therapy arising out of the method for determining the predominant physiological effect of a composition comprising hemoglobin, if the predominant physiological effect of the composition is vasodilation, then the patient in need of nitric oxide biological activity can be administered added thiol, for example, by administering to a human patient *N*-acetylcysteine IV at 50-100 mg/kg or PO at 600 mg 3 times per day.

Yonetani Takashi et al., J. Biol. Chem. 273(32): 20323-20333 (1998) relates to electron paramagnetic resonance and oxygen binding studies of α-nitrosyl hemoglobin.

### BRIEF DESCRIPTION OF THE DRAWINGS

Figure 1A is a graph showing EPR spectra of iron nitrosyl-Hb derivatives as described in Example 1.
Figure 1B is a graph showing EPR spectra of iron nitrosyl-Hb derivatives as described in Example 1.
Figure 1C is a graph of HbNO yield (in 10 mM phosphate) as a function of O₂ saturation, as described in Example 1.
Figure 1D is a graph of HbNO yield (in 100 mM phosphate) as a function of O₂ saturation, as described in Example 1.
Figure 2 is a graph of metHb yield as a function of O₂ saturation, as described in Example 2.
Figure 3A is a graph showing nitrosyl yield as a function of Hb concentration, as described in Example 3.
Figure 3B shows difference spectra of metHb (solid line), deoxyHb (dotted line), and iron nitrosylHb (dashed line) vs. oxyHb, as described in Example 3.
Figure 3C shows difference spectra generated from the exposure of NO to normoxic (≈99% oxygen saturated) Hb, as described in Example 3.
Figure 3D shows calculated fits for difference spectra shown in Fig. 3C, as described in Example 3.
Figure 4A is a graph of nitrosyl yield versus hemoglobin, showing the effect of SOD, as described in Example 4.
Figure 4B is an EPR spectrum of a DNIC formed by exposure of oxyHb (≈99% saturated; 3.93 mM) to NO (36 µM). See Example 4.
Figure 4C is a bar graph showing formation of *S*-nitroso-hemoglobin and iron nitrosyl-hemoglobin formed by exposure of oxyHb to NO. See Example 4.
Figure 4D is a bar graph showing formation of intraerythrocytic S-nitroso-Hb and iron nitrosylHb in oxygenated RBCs. See Example 4.
Figure 5 shows EPR spectra as described in Example 5.
Figure 6 shows EPR spectra as described in Example 5.
Figures 7A-7C are bar graphs showing the distribution in cytosolic and membrane fractions of NO groups following exposure of intact RBCs to NO.

A: Recovery of NO is essentially complete at low, physiological NO:heme ratios, which yield 100-800 nM intracelluar NO.
B: Fe(II)NO is predominantly cytosolic.
C: SNO is largely membrane-associated (p<0.05 for all pairwise comparisons). Hb-derived SNO is associated with cysteine thiols of RBC membrane proteins.

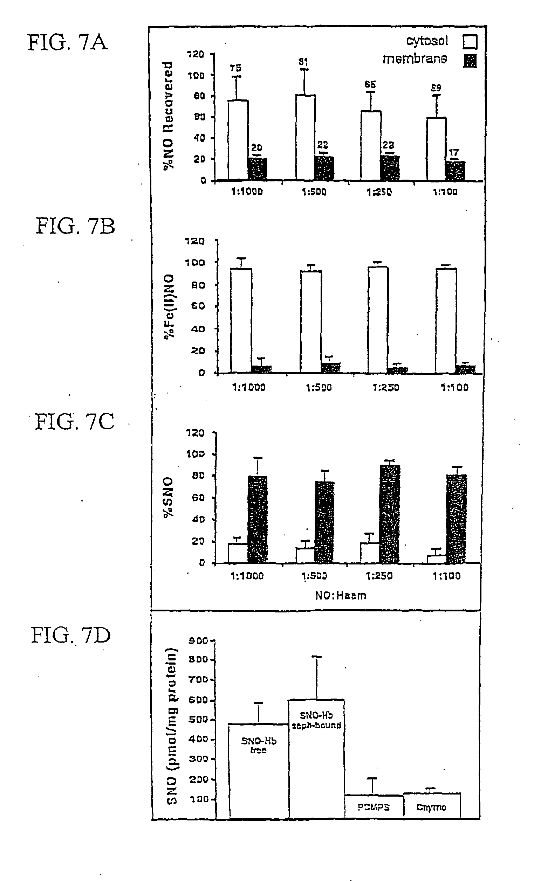

Figure 7D is a bar graph showing SNO content of IOV extracts following incubation with free or Sepharose-bound SNO-Hb (50 nmoles SNO-Hb/mg IOV protein). Transfer of NO groups to the membrane is greatly reduced (p<0.05) following treatment of IOVs with the thiol-modifying reagent PCMPS and following mild digestion of IOVs with chymotrypsin (chymo). (n = 3-7 for a-d).

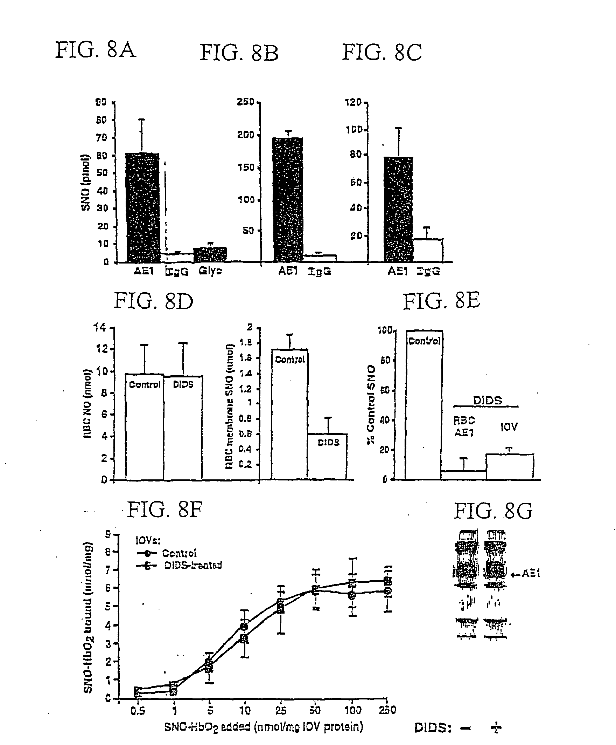

Figures 8A-8C are bar graphs demonstrating that AE1 is *S*-nitrosylated by SNO-Hb in intact RBCs and IOVs. SNO content of IPs derived from IOVs incubated with (A) free or (B) Sepharose-bound SNO-Hb or from (C) membrane extracts of RBCs treated with NO (NO:heme ratio of 1:250). IPs were generated with monoclonal antibodies specific for AE1 or glycophorin (Glyc) or with a non-specific mouse IgG.

Figures 8D and 8E are bar graphs demonstrating specific inhibition of transnitrosylation by the AE1 inhibitor DIDS (0.1 mM). (D) Prior treatment with DIDS does not reduce total NO (SNO plus Fe[II]NO) incorporated by RBCs (NO:heme ratio of 1:250), but substantially decreases membrane SNO content. (Samples were derived from 2.5 X 10⁹ RBCs and thus contained 4 nmoles of AE1). (E) SNO content is greatly reduced compared to DIDS-free controls both in IPs of AE1 from membrane extracts of RBCs treated with DIDS before exposure to NO (as in C) and in extracts of IOVs derived from DIDS-treated RBCs and incubated with free SNO-Hb (as in Figure 7D) (p<0.05).

Figure 8F is a graph showing SNO-oxyhemoglobin bound to IOVs with increasing added SNO-oxyhemoglobin. SNO-Hb binds with equal affinity to IOVs derived from native or DIDS-treated RBCs.

Figure 8G is a fluorographic image of proteins on an SDS-polyacrylamide gel following electrophoresis. DIDS does not directly reduce reactivity of RBC membrane thiols as assessed by alkylation with ¹⁴C-iodoacetamide of extracts of IOVs derived from native or DIDS-treated RBCs, followed by SDS-PAGE and fluorography. The position of AE1 on the gel, determined by Western blotting, is indicated. (n = 3-8 for A-F).

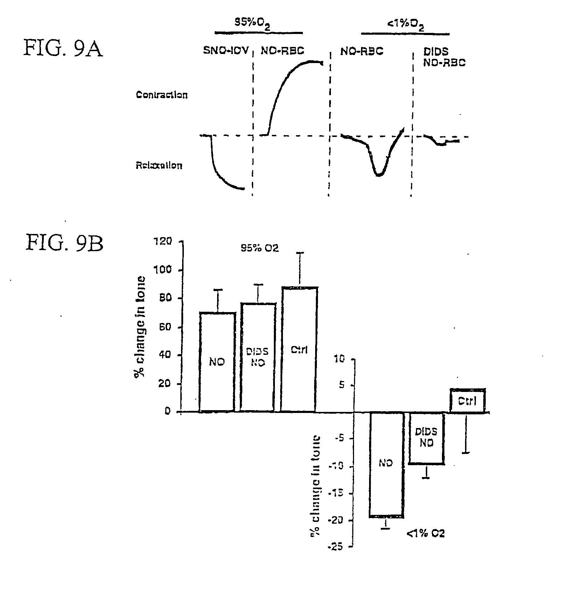

Figure 9A shows representative polygraph traces of the tension developed by aortic ring segments in bioassay medium of specified pO₂. At 95% O₂, relaxation follows addition of SNO-IOVs (60 nM final SNO concentration), while addition of NO-treated RBCs elicits contraction. At <1% O₂, addition of NO-treated RBCs (containing 60 nM SNO) elicits relaxation, while addition of RBCs treated with DIDS before exposure to NO has little effect.

Figure 9B is a bar graph which summarizes the bioassay results such as those shown in Figure 9A (n = 5-7) displaying changes in aortic tension elicited at 95% O₂ or <1% O₂ by addition of RBCs previously exposed to physiological amounts of NO (NO:heme ratio of 1:250; final SNO concentration of 60 nM), RBCs treated with DIDS before exposure to NO, and control RBCs deoxygenated and reoxygenated as for NO treatment (for DIDS-NO at 1% pO₂, p<0.05 vs. NO but not significantly different from control).

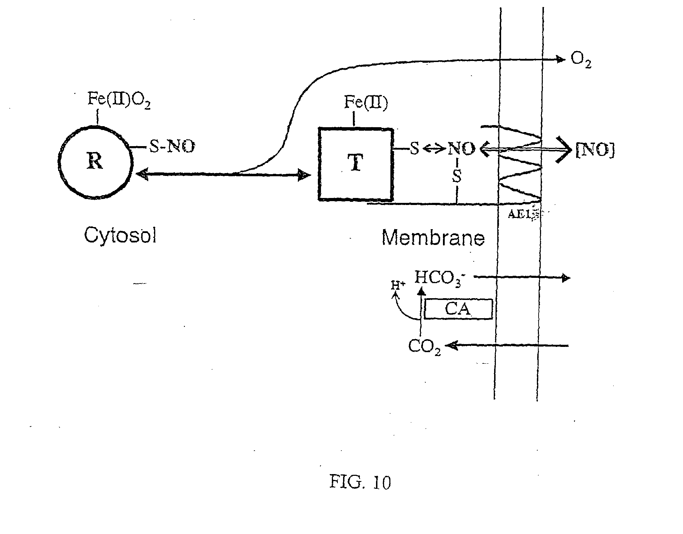

Figure 10 is a diagram showing the pathway for export from the RBC of NO-related bioactivity via transfer of NO groups from cysβ93 of Hb at the membrane-cytosol interface.

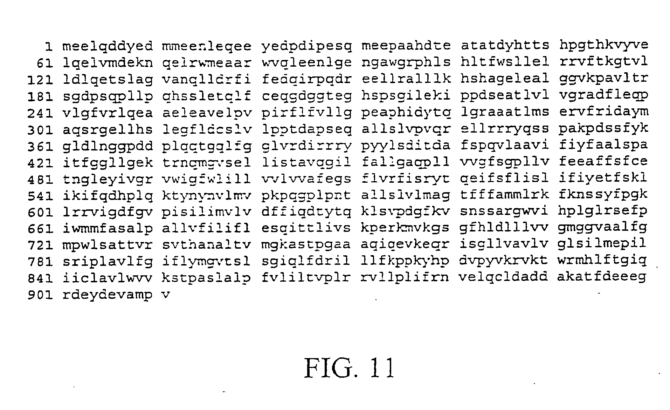

Figure 11 is a representation of the amino acid sequence (SEQ ID NO:2) of band 3 anion transport protein from human erythrocytes. See GenBank Accession No. 2144877.

### DETAILED DESCRIPTION OF THE INVENTION AND THE DISCLOSURE

### Abbreviations

Hb, hemoglobin; SNO, *S*-nitrosothiol; oxyHb, oxyhemoglobin; metHb, methemoglobin; deoxyHb, deoxyhemoglobin; nitrosylHb, nitrosylhemoglobin; DNIC, dinitrosyl iron complex; RBC, red blood cell; UV ultraviolet; DTPA, diethylene triamine pentaacetic acid; EDTA, ethylene diamine tetraacetic acid; PBS, phosphate buffered saline; RBCs, red blood cells; IOV, inside-out vesicles; IPs, immunoprecipitates; SOD, superoxide dismutase; PCMPS; p-chloromercuriphenylsulfonic acid; EPR, electron spin resonance spectroscopy; G6PD, glucose-6-phosphate dehydrogenase.

The chemistry of nitric oxide (NO) interactions with Hb has served as a ubiquitous model within the field of NO biochemistry. For example, the oxidative interaction of NO with oxyhemoglobin (oxyHb) to produce nitrate is considered to be the major route of NO catabolism (Kelm, M., et al., pp. 47-58 in Metabolic Fate of Nitric Oxide and Related N-Oxides, eds. Feelisch, M. & Stamler, J.S., Wiley, London 1st Ed., 1996; Pietraforte, D., et al., Biochemistry, 34:7177-7185 1995; Wennmalm A., et al., Br. J. Pharmacol, 106:507-508, 1992) as well as a reliable method for assaying NO (Feelisch, M., et al., pp. 455-478 in The Oxhemoglobin Assay, eds. Feelisch, M. & Stamler, J.S., Wiley, London, 1st Ed., 1996); likewise the unique ability of NO to induce displacement of a trans-imidazole heme ligand, has been proposed as key to its activation of guanylyl cyclase (Traylor, T.G., et al., Biochem., 31:2847-2849, 1992). In the specific realm of the cardiovascular system, these reactions: are fundamental elements of models for NO diffusion (Liu, X., et al., J. Biol. Chem., 273:18709-18713, 1998; Lancaster, J.R., Jr., Proc. Natl. Acad. Sci. USA, 91:8137-8141, 1994); played a crucial role in the identification of endothelium derived relaxing factor (Liu, X., et al., J. Biol. Chem., 273:18709-18713, 1998; Lancaster, J.R., Jr., Proc. Natl. Acad. Sci. USA, 91:8137-8141, 1994; Palmer, R.M., et al., Nature (London), 327:524-526, 1987; Ignarro, L.J., et al., Proc. Natl. Acad. Sci. USA, 84:9265-9269, 1987); and inform a variety of therapeutic applications, including NO-inhalation therapy (Roissant, R., et al., New Eng. J. Med., 328:399-405, 1993; Wessel, D.L., et al., Circulation, 88:2128-2139, 1993) and blood substitute design (Alayash, A.I., et al., Mol. Med. Today, 1:122-127, 1995; Doherty, D.H., et al., Nature Biotechnol., 16:672-676, 1998).

Measurements of the rates of these reactions show that the NO-mediated oxidation of oxyHb to methemoglobin (metHb) is kinetically competitive with the binding of NO to unoccupied hemes in Hb -- with specific rate constants of 3.7 x 10⁷ M⁻¹ sec⁻¹ and 2.6 x 10⁷ M⁻¹ sec⁻¹, respectively (Cassoly, R., et al., J. Mol. Biol., 91:301-313, 1975; Doyle, M.P., et al., J. Inorg. Chem., 14:351-358, 1981; Eich, R.F., et al., Biochemistry, 35:6976-6983, 1996). The rates of NO oxidation of oxymyoglobin and NO binding to ferrous myoglobin are also very similar (3.4 x10⁷ M⁻¹ sec⁻¹ vs 2.5 x10⁷ M⁻¹ sec⁻¹) (Eich, R.F., et al., Biochemistry, 35:6976-6983, 1996). Such a rapid route of NO metabolism is, however, difficult to reconcile with mammalian NO production rates (Castillo, L., et al., Proc. Natl. Acad. Sci. USA, 93:11460-11465, 1996), which are orders of magnitude too low to sustain physiological NO levels (10 nM-1 µM) (Lancaster, J.R., Jr., Proc. Natl. Acad. Sci. USA, 91:8137-8141, 1994; Pinsky, D.J., et al., Circ. Res., 81:372-379, 1997; Vallance, P., et al., Lancet, 346:153-154, 1995; Jia, L., et al., Nature (London), 380:221-226, 1996), were NO to be freely consumed in these reactions.

The measured NO synthesis rate is 1.3 millimoles per day for the average person (Castillo, L., et al., Proc. Natl. Acad. Sci. USA, 93:11460-11465, 1996). To maintain a basal NO concentration of 10 nM - 1 µM *in vivo* (Liu, X., et al., J. Biol. Chem, 273:18709-18713, 1998; Lancaster, J.R., Jr., Proc. Natl. Acad. Sci. USA, 91:8137-8141, 1994; Pinsky, D.J., et al., Circ. Res., 81:372-379, 1997; Vallance, P., et al., Lancet, 346:153-154, 1995; Jia, L., et al., Nature (London), 380:221-226, 1996), 130 to 13,000 moles of NO would be consumed per day in reaction with Hb (assuming Kₒₓ = 3.7 x 10⁷M⁻¹ sec⁻¹ (Cassoly, R., et al., J. Mol. Biol., 91:301-313, 1975), 5 L vascular volume). The hypothetical NO consumption rate, therefore, is 10⁵- to 10⁷-fold greater than the actual production rate.

Previous studies of the NO oxyHb reaction, however, had been performed with NO concentrations 10-fold greater than protein (Eich, R.F., et al., Biochemistry, 35:6976-6983, 1996). Under physiological conditions, the concentration ratio is starkly different, with NO concentrations 1000-fold lower than Hb (Jia, L., et al., Nature (London), 380:221-226 (1996). Moreover, there is always a population of heme sites that are unoccupied. In highly oxygenated Hb, as found in arterial blood, this population is small (≈ 1%) but is nevertheless in excess of NO. The influence of these vacant hemes, in the physiological situation, cannot be ignored; they might successfully compete for NO with the much larger fraction (≈99%) of oxygen-ligated hemes, if NO binding to hemes in oxyHb were cooperative, that is, if NO addition rates were to increase with increasing oxygen saturation. This possibility has not been raised in previous discussions of NO and Hb chemistry (Kelm, M. & Yoshida, K., pp. 47-58 in Metabolic Fate of Nitric Oxide and Related N-Oxides, eds. Feelisch, M. & Stamler, J.S., Wiley, London, 1st Ed., 1996; Pietraforte, D., et al., Biochemistry, 34:7177-7185, 1995; Wennmalm, A., et al., Br. J. Pharmacol, 106:507-508, 1992; Feelisch, M., et al., pp. 455-478 in The Oxhemoglobin Assay, eds. Feelisch, M. & Stamler, J.S., Wiley, London, 1st Ed., 1996; Traylor, T.G., et al., Biochem., 31:2847-2849, 1992; Liu, X., et al., J. Biol. Chem., 273:18709-18713, 1998; Lancaster, J.R., Jr., Proc. Natl. Acad. Sci. USA, 91:8137-8141, 1994; Eich, R.F., et al., Biochemistry, 35:6976-6983, 1996; Marletta, M.A., et al., Biofactors, 2:219-225, 1990; Moore, E.G., et al., J. Biol. Chem., 251:2788-2794, 1976; Antonini, E., et al., p. 13 in Frontiers in Biology, Neuberger, A. and Tatum, E.L., eds., North-Holland Publishing Co., Amsterdam, London, 1971; Sharma, V.S., et al., Biochemistry, 26:3837-3843, 1987). On the contrary, the demonstrated lack of cooperativity in the binding of NO to deoxyhemoglobin (deoxyHb) (Cassoly, R., et al., J. Mol. Biol., 91:301-313, 1975) -- which indicates that the intrinsic NO addition rate constants do not change with *NO saturation* -- implicitly shapes the current perspective. It is important to recognize, however, that these results do not imply that the NO addition rates to oxygenated Hb are similarly independent of the *oxygen saturation,* and thus cannot be assumed to apply to the physiological situation. In addition to these oxidation and addition reactions, recent studies (Jia, L., et al., Nature (London), 380:221-226, 1996; Gow, A.J., et al., Nature (London), 391:169-173, 1998; Stamler, J.S., et al., Science, 276:2034-2037, 1997), make it clear that additional reactions, in particular S-nitrosylation, should be considered in any assessment of the chemical interplay of NO and human Hb. The *S*-nitrosylation reaction assumes particular importance inasmuch as it conserves, rather than consumes, NO bioactivity.

Herein are described reactions that occur on exposure of Hb to NO at relative concentrations that reflect the physiological situation. Applicants show that the addition of NO to oxyHb takes advantage of the cooperative effects of oxygen binding and thus effectively competes with the oxidation reaction. It is further described that at high oxygen saturations, reactions that *S*-nitrosylate the protein occur to a significant extent. Taken as a whole, these data indicate that the interaction of NO with oxyHb, rather than destroying NO bioactivity as widely misapprehended, acts to preserve it -- that Hb very cleverly introduces new chemistry, when oxygen saturation is high, that limits oxidation and channels the NO groups into products that preserve their bioactivity. This picture represents a substantial reversal of the conventional thinking on the chemistry of Hb as it pertains to NO biology and has fundamental implications for the general chemistry of heme-containing proteins.

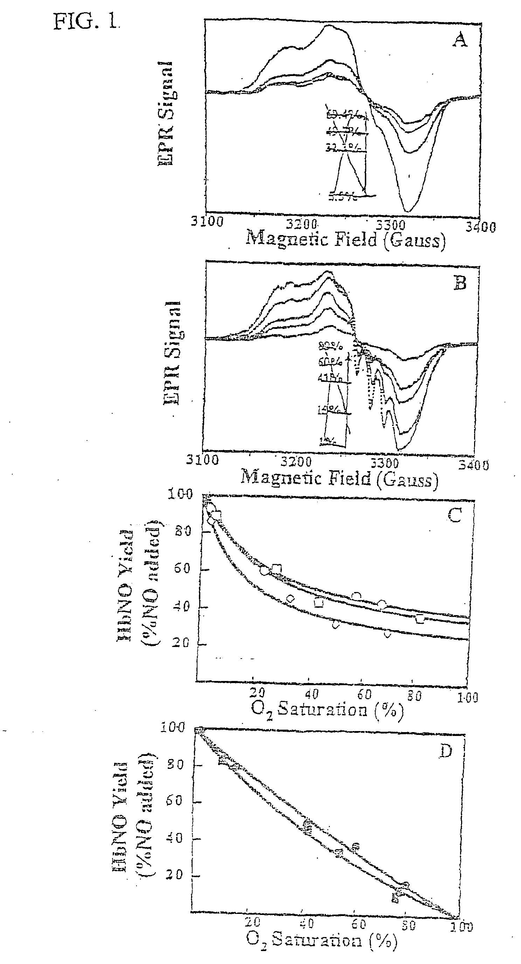

EPR spectroscopy was used to assess the formation of nitrosyl heme on addition of NO to Hb preparations with oxygen saturations (Y) in the range 0-80% (typical EPR spectra, are shown in Figures 1A and B). EPR signal intensities were used to quantify the proportion of nitrosylated hemes relative to the NO initially added; the results of this quantification are plotted vs. Y in Figure 1C (10 mM phosphate) and D (100 mM phosphate). The data obtained at high phosphate levels follow the behavior described by Eq. 4, the solid curves through the data points are graphs ofEq. 4 with κ values for the two depicted curves averaging 1.40 ± 0.06. The data obtained at the lower phosphate level, however, exhibit a notable deviation from the simple model: they cross the diagonal, thus showing a progressive overproduction of nitrosyl heme. Furthermore, the limiting tangential slopes indicate that κ is decreasing with increasing Y. By empirical curve fitting, we found that the data in Figure 1C are well described by a function of the form (1 + Y)/(1 + cY) (c, a constant). (The solid lines in Figure 1C are graphs of this function with least-squares best values of the parameter c). This functional form can be assimilated to that of Eq. 4, provided κ is allowed to vary with Y (specifically, κ = (c - 1)(1 - Y)/(1 + Y)]. This result indicates that over the 0 to 80% range of oxygen saturation, κ decreases 7-fold and suggests, by extrapolation, a 100-fold decrease at 90% saturation. We attribute this variation in κ primarily to an increase in k_{add}, as kₒₓ does not vary by more than a factor of 2, as judged from the limiting slopes, and the literature values for kₒₓ (Y = 100%) and k_{add} (Y = 0%).

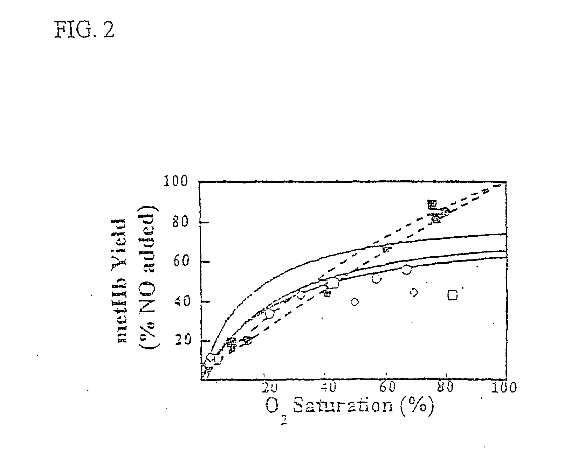

We also assessed the formation of oxidized ferric hemes with EPR (data not shown) and UV-visible difference spectroscopy (Figure 2). The results obtained from samples in 100 mM phosphate conform to the simple competition model: the dashed lines in the figure, which are calculated from the curves depicted in Figure 1D following Eq. 5, agree extremely well with the experimental measurements. Experiments conducted in 10 mM phosphate, however, show a stark deviation from the simple model behavior. Qualitatively, the results show that heme oxidation never grossly exceeds heme-nitrosylation. Moreover, there is a progressive shortfall in the Fe(III) and Fe(II)NO products. This shortfall is indicated by the departure of the experimental points (10 mM phosphate) in Figure 2, from the curves calculated from the curves depicted in Figure 1D following Eq. 5, and amounts to as much as ≈20% of added [NO]₀. This behavior strongly suggests the presence of additional NO reaction pathways.

In summary, NO binding to oxyHb is cooperative; oxidation to ferric heme (metHb) is limited under physiological conditions; additional chemistry is occurring in the more oxygenated Hb species that are prevalent *in vivo.* These findings might seem at odds with previous literature suggesting that NO binding Hb is non-cooperative (Cassoly, R., et al., J. Mol. Biol., 91:301-313, 1975). The proper conclusion to draw from these prior studies, however, is that NO (*ligand*) binding to *nitrosylHb* shows little cooperativity with varying NO-saturation -- a scenario of little physiological relevance, because NO is never the dominant ligand *in vivo.* Our results reflect the physiological situation in which the ligand, NO, binds to Hb with some degree of oxygen saturation. The functional behavior in this situation is, not surprisingly, cooperative. In this regard, experiments of particular interest are those conducted in the presence of high phosphate concentrations (100 mM), which perturb the allosteric modulation of ligand affinity by disfavoring the relaxed [R (oxy)] structure among the partially ligated hemoglobins, as evidenced by the hyperfine structure in the EPR (Figure 1B) (Takahashi, Y., et al., Am. J. Physiol., 274:H349-H357, 1998). Thus, normal tight [T (deoxy)]/R (oxy) interconversion in Hb appears to be essential for "normal" NO function (Figures 1A and C). Taken together, the EPR results demonstrate that when the oxygen-induced allosteric transition is unhindered, NO binding to oxygenated Hb is cooperative -- a situation that leads to enhanced iron nitrosyl- and limited metHb formation.

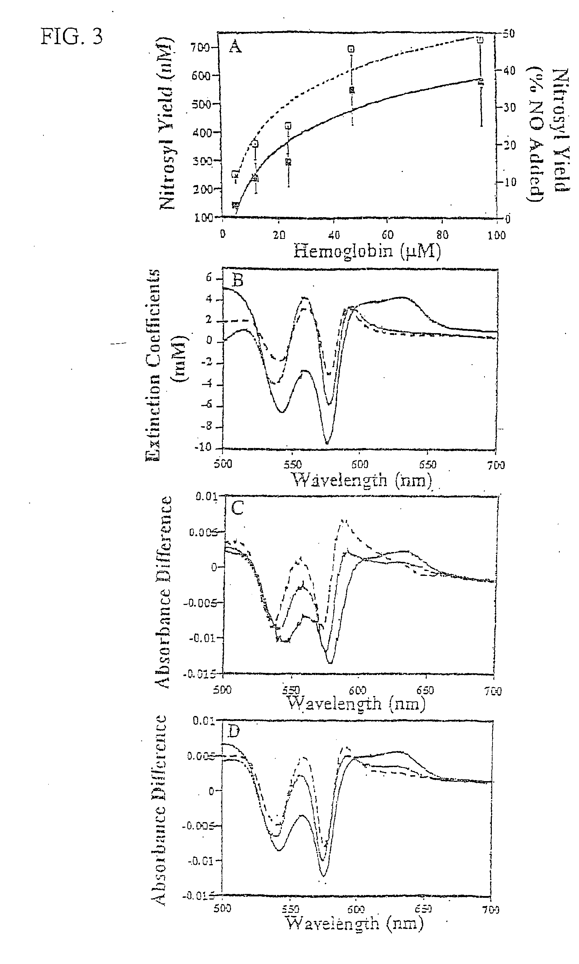

To extend these results to arterial oxygen saturation (of =99%) and physiological NO concentrations (≈0.3 µM), we employed photolysis-chemiluminescence to measure nitrosyl derivatives of Hb (Figure 3A). In these experiments, normoxic Hb is in excess of NO, but NO is in excess of the vacant hemes, a scenario disfavoring NO addition: Our results show that even at high oxygen saturation, a substantial fraction of the NO -- rather than forming nitrate by the oxidation reaction -- forms chemiluminescence-detectable nitrosyl derivatives. Of further interest, the yield of nitrosyl species increases with increasing [Hb] up to a maximum of approximately 50% of NO added (relative to the [Hb] the nitrosyl yield varies from 3 to 0.6%) (Figure 3A) In the simple competition model, the fraction of nitrosylation products would be independent of protein concentration. These results thus clearly demonstrate that additional reactions, beyond NO binding to vacant hemes to form the nitrosyl-heme derivative, are occurring under these conditions.

To gain further insight into this chemistry, we used the discriminating power of difference absorption spectroscopy. Difference spectra obtained by titration of submicromolar concentrations of NO against 33 µM Hb in room air (99% O₂ saturation) are shown in Figure 3C; standard difference spectra of authentic met-, deoxy- and nitrosylHb relative to oxyHb are shown in Figure 3B. If the chemistry were to proceed according to the simple model, then at Y = 99% the oxidation reaction would predominate and the observed difference spectra would closely resemble the metHb minus oxyHb standard difference spectrum. This behavior was observed only at high phosphate concentrations (Figure 3C and D), consistent with the EPR results above. At low phosphate concentrations, we found that the difference spectra point largely toward the formation of nitrosylated heme: much of the difference spectrum can be accounted for by the deoxyHb minus nitrosylHb standard spectrum (Figure 3C). To produce adequate difference-spectrum simulations, it was necessary to include a deoxyHb minus oxyHb component (presumably reflecting compensation for the nitrosylative loss of vacant hemes), and, most significantly, to relax the mass-balance constraint: a measurable fraction of [NO]₀ was not accounted in the Hb spectra (Figures 3C and 3D); the spectra account for *only* 50% in low phosphate and 80% in low phosphate plus borate. Taken as a whole, these data extend to normoxic conditions the conclusions made above, namely: direct oxidation by NO is not the predominant reaction at low NO to heme ratios; addition of NO to vacant hemes remains competitive; and further reaction pathways, beyond oxidation and addition, must be occurring.

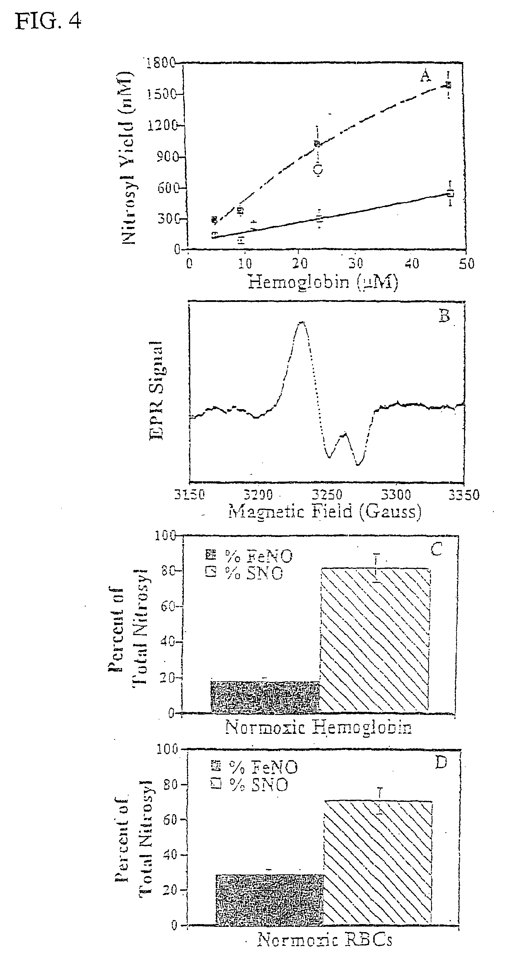

One additional species that could compete for NO is superoxide -- liberated by the autooxidation of oxygenated Hb (Misra, H.P., et al., J. Biol. Chem., 247:6960-6962, 1972). To examine this possibility, we repeated the experiments detailed in Figure 3A in the presence of superoxide dismutase (SOD) (Figure 4A). At all concentrations of Hb used, the presence of SOD increased the yield of Hb nitrosyl derivatives (i.e., total NO bound) to approximately 100% of [NO]ₒ (Figure 4A). Similarly, SOD led to increases in the yield of nitrosylated hemes detected in the EPR experiments (Figure 1 C). Evidently, under these conditions, superoxide is a significant competitor for NO, or perhaps alters the reactivity (oxidation and/or ligand binding) of oxygenated Hb. When these experiments were performed with stroma-free Hb, a RBC preparation that contains normal levels of SOD, similar results were observed (Figure 4A). It is further notable, that analogous effects on the nitrosyl yield -- assessed by EPR, chemiluminescence, and difference spectroscopy -- were obtained when borate was included in the buffer medium (Figures 1A, 2 and 3C). Borate most likely exerts this effect by altering the ligand on-rate for NO or the reactivity of the oxygen ligand with NO, or perhaps the intrinsic autooxidation rate of Hb. Phosphate levels may also influence these parameters.

An important clue to additional reaction pathways comes from our analyses under normoxic conditions: nitrosyl yields as high as 6% of Hb were observed by photolysis-chemiluminesence, notwithstanding the fact that the proportion of heme vacancies is only ≈ 1%. These nitrosyl species, moreover, did not affect the UV-visible spectra. EPR of samples under these conditions exhibit spectra similar to the DNICs (DNICs) exhaustively studied by Vanin (Vanin, A.F., et al., Nitric Oxide, 1:191-203, 1997), albeit they account for a small percentage of NO added (Figure 4B). DNICs are known to form from SNOs with which they exist in equilibrium (Vanin, A.F., et al., Nitric Oxide, 1:191-203, 1997). Indeed, chemiluminesence analysis of the products formed upon addition of 1.2 µM NO to 48 µM oxyHb, which produces nitrosyl yields of approximately 500 nM (Figures 1A and 3A), show that ≈80% of this nitrosyl yield is SNO (Figure 4C). Moreover, treatment of aerated RBCs with physiological concentrations of NO (0.3 µM) resulted in relatively high yields on intracellular *S*-nitroso-oxyHb (Figure 4D). Specifically, analyses revealed (after inherent time delays of ≈30 min.) yields of intracellular S-nitrosoHb, iron-nitrosylHb, and metHb of 103 ± 38 nM, 42 ± 15 nM, and 0 nM (i.e., none detectable), respectively (n = 12), and the further appearance of nitrosyl heme adducts upon lowering of the oxygen tension, in general agreement with studies on isolated Hb (Figures 1, 3A, 4A, and 4C).

Although the oxidation reaction (Eq. 1) has been given great significance in NO biology, our data demonstrate that it is likely to be of little significance under normal physiological conditions. Because of the low concentration of NO relative to Hb, vacant hemes are in excess over NO. This excess, together with the cooperativity of ligand binding in oxyHb, enables the addition of NO to heme to compete with the oxidation reaction even at high oxygen saturation. Moreover, in oxygenated Hb, additional reactive pathways *that preserve NO bioactivity* are available, including the production of SNO and DNIC. These results are in keeping with *in vivo* observations ofHb nitrosyl derivatives, the levels of which are generally unrelated to metHb concentration (Takahashi, Y., et al., Am. J. Physiol., 274:H349-H357, 1998), directly responsive to NO administration (Takahashi, Y., et al., Am. J. Physiol., 274:H349-H357, 1998), and dynamically controlled by allosteric state of Hb (Stamler, J.S., et al., Science, 276:2034-2037, 1997; Hall, D.M., et al., J. Appl. Physiol., 77:548-553, 1994) but otherwise unrelated to Hb oxygen saturation (Stamler, J.S., et al., Science, 276:2034-2037, 1997; Takahashi, Y., et al., Am. J. Physiol., 274:H349-H357, 1998; Hall, D.M., et al., J. Appl. Physiol., 77:548-553, 1994). These findings also help reconcile NO biochemistry with NO production rates in mammals (Castillo, L., et al., Proc. Natl. Acad. Sci. USA, 93:11460-11465, 1996) -- which are orders of magnitude too low to sustain physiological NO levels, were the oxyHb reaction dominant. In addition, they rationalize the ability of inhaled NO, which is purportedly inactivated by Hb in the lungs (Roissant, R., et al., New Eng. J. Med., 328:399-405, 1993; Wessel, D.L., et al., Circulation, 88:2128-2139, 1993; Westfelt, U.N., et al., Br. J. Pharmacol., 114:1621-1624, 1995), to lower systemic blood pressure (Wessel, D.L., et al., Circulation, 88:2128-2139, 1993), increase aortic tissue cGMP levels (Kermarrec, N., et al., Am. J. Respir. Crit. Care Med., 158:833-839, 1998), avert sickling of RBCs (Head, C.A., et al., J. Clin. Invest., 100:1193-1198, 1997), improve blood flow to ischemic tissues (Fox-Robichaud, A., et al., J. Clin. Invest., 101:2497-2505, 1998), and increase glomerular filtration rate (Troncy, E., et al., Br. J. Anaesth., 79:631-640, 1997).

These discoveries have a strong bearing both on the way NO heme interactions are modeled and our understanding of NO biology. The current view of the NO interaction with Hb *in vivo* is derived from a model in which the elimination as NO₃⁻ is dominant, and NO release from Hb is inconsequential (Kelm, M., et al., pp. 47-58 in Metabolic Fate of Nitric Oxide and Related N-Oxides, eds. Feelisch, M. & Stamler, J.S., Wiley, London, 1st Ed., 1996; Wennmalm, A., et al., Br. J. Pharmacol, 106:507-508, 1992; Feelisch, M., et al., pp. 455-478 in The Oxhenioglobin Assay, eds. Feelisch. M. & Stamler, J.S., Wiley, London, 1st Ed., 1996; Liu, X., et al., J. Biol. Chem., 273:18709-18713, 1998; Lancaster, J.R., Jr., Proc. Natl. Acad. Sci. USA, 91:8137-8141, 1994; Doyle, M.P., et al., J. Inorg. Chem., 14:351-358, 1981; Eich, R.F., et al., Biochemistry, 35:6976-6983, 1996; Sharma, V.S., et al., J. Biol. Chem., 253:6467-6472, 1978). In reality, Hb musters additional reaction pathways to keep the balance in favor of maintaining the NO group in a bioactive state. These chemical reactions with thiols, metals and superoxide are the essential elements of the extended paradigm of NO biochemistry presented some years ago (Stamler, J.S., et al., Science, 258:1898-1902, 1992).

Our results have important implications for rational design of blood substitutes, NO scavengers and therapeutic NO donors. Additionally, they predict that measurements of NO with the oxyHb assay will tend to underestimate NO production, unless appropriate precautions are taken, and more generally point to limitations of Hb-based approaches for identification of NO bioactivity. Finally, these findings raise fundamental questions. For example, nitrate remains the major metabolic product of NO *in vivo*, but the question now arises as to its source. It is tempting to suggest the involvement of a heme protein that can neither enforce the cooperativity of ligand binding, nor recruit the thiol reaction pathway. These properties are exemplified in the bacterial flavohemoglobin whose recently identified enzymatic function involves the oxidation of NO to nitrate (Gardner, P.R., et al., Proc. Natl. Acad. Sci. USA, 95:10378-10383, 1998; Hausladen, A., et al., Proc. Natl. Acad. Sci. USA, 95:14100-14105, 1998). Whereas the primordial bacterial Hb is designed to metabolize NO (Hausladen, A., et al., Proc. Natl. Acad. Sci. USA, 95:14100-14105, 1998), mammalian Hb is designed to secure and deliver it, (Gow, A.J., et al., Nature (London), 391:169-173, 1998; Stamler, J.S., et al., Science, 276:2034-2037, 1997). These observations suggest that the molecular evolution of Hb was impacted by its NO-related functions.

Hemoglobin exists in two alternative structures: one with high affinity for oxygen, termed R or oxy, and the other with low affinity, termed T or deoxy. It is the switch from the T structure to the R structure that is the basis for cooperativity in oxygen binding. This theory predicts that most molecules in the T or deoxy structure will have no oxygen molecules bound, whereas the majority of molecules in the R or oxy structure will have 4 oxygen molecules bound. The theory also predicts the existence of minor populations of molecules in T or R structure that will be partially ligated (Eaton, W.A., Nature Struc. Biol. 6:351-358, 1999). For example, oxyhemoglobin in room air is approximately 99% oxygen saturated and 1% deoxygenated. This 1% of deoxygenated hemes can exist as 1% tetramer with no oxygen bound or 4% tetramer with 3 oxygens bound or as a mixture of populations with 1, 2, or 3 molecules bound. These vacancies in hemoglobin, generally constituting a very minor fraction of the total population of hemoglobin molecules, have been assumed to not have any functional importance. That is, it has been assumed that it makes no difference from the standpoint of oxygen delivery whether 1% of Hb molecules carry no oxygens or if 2% each carry 2, or if 4% carry 1.

It is shown herein 1) that the distribution of the hemoglobin population having vacancies on the hemes controls the function of hemoglobin; 2) by regulating the functional behavior of this vacancy population, hemoglobin can either a) quench and eliminate excess nitric oxide (Equation 1) or b) store excess nitric oxide in a form that is not a donor of NO, or c) store NO in a form that donates NO (or in a form that can be readily transformed into a SNO donor of NO or a dionitrosyl iron complex donor of NO).

The adverse properties of hemoglobin-based substitutes and related therapeutics results in significant part from reactions with nitric oxide (See Alayash, A.I., Nature Biotechnoloy 17: 545-549, 1999). On the other hand, no means exists to ensure that hemoglobin will channel the NO into bioactive SNO for therapeutic applications. Herein is described a strategy for both eliminating undesirable effects of hemoglobins and for channeling NO into desirable SNO by controlling the distribution of vacancies in hemoglobin. Methods are also described by which it is possible to eliminate NO where overproduction is toxic or, alternatively, store it in an inaccessible reservoir when elimination has undesirable effects (either due to oxidative chemistry or because some NO is required).

The basic tenets are as follows. 1) For storage of NO (in non-donor form, as iron nitrosylhemoglobin): maximize vacancies of molecules in R structure (e.g., maximize R3 state) while minimizing oxyligated hemes on hemoglobins in T state (e.g. T1). 2) For quenching of NO (that is, consumption of NO by Hb and oxidizing NO to nitrate with formation of metHb): minimize vacancies of molecules in R structure (e.g., R3) whilst maximizing oxyhemes of molecules in T structure (e.g., T1). 3) For SNO and DNIC formation (i.e., storage in bioactive form as donors of NO) the requirements are both 1) above and in addition it is required that the vacancies can undergo redox chemistry, without which NO cannot transfer from heme to thiol. "Redox chemistry" refers to the transfer of NO from the heme Fe to cysteine on the P subunit with the loss of an electron. See, for example, WO 98/34955 regarding the conversion of iron nitrosyl-hemoglobin to SNO-hemoglobin.

Herein is described a test for a composition comprising any type of hemoglobin for these properties or the effect of any solution or allosteric effector on hemoglobin function *in vivo*. The test involves determination of the product distribution among nitrosyl hemoglobin, methemoglobin and SNO hemoglobin as a function of oxygenation in hemoglobin, when NO is added to hemoglobin as a limiting reagent (as shown in Figures 1A-1D). EPR and/or oxygen binding curves can be used to predict those buffers which promote the vasoconstrictor activity of hemoglobin and those that will promote vasodilation. In the simple competition model, cooperativity of NO binding identifies tenet 1, whereas lack of cooperativity identifies tenet 2. In addition, it is demonstrated that cooperativity of NO binding is not sufficient for transformation of NO into bioactive form (see Figure 3C and Figure 3D, borate). Thus, by regulating the auto-oxidation function of hemoglobin in vacancies (e.g., 10 millimolar phosphate vs. borate) or by adding redox modifiers such as nitrite, one can greatly enhance the transformation into SNO or DNIC.

Examples of solutions that alternatively achieve storage, quenching, or SNO formation are provided. Specifically, hemoglobin solutions in 10 millimolar phosphate plus 90 millimolar borate show cooperatively of NO binding to heme Fe in the absence of efficient SNO formation. That is, this solution traps and stores NO. In contrast, 10 millimolar phosphate alone results in cooperativity of NO binding and high yield of SNO formation; that is, transformation of NO into SNO is achieved by preserving redox chemistry in hemoglobin. Lastly, solutions of 100 millimolar phosphate result in lack of cooperativity of NO binding and thus in quenching of NO (tenet 2).

The following are examples of some expected applications of hemoglobin compositions in methods of therapy.

In a patient with septic shock who develops severe myocardial depression; pancreatitis and progressive respiratory failure, it can be concluded that nitric oxide overproduction has likely contributed to organ failure. The patient can be treated with an intravenous infusion of hemoglobin 100 milligrams per kilogram in a composition containing 100 millimolar phosphate to decrease circulating NO and convert it to nitrate.

A patient with septicemia as a complication of a urinary tract infection receives an intravenous infusion of phenylephrine to maintain systemic blood pressure in the normal range. However, the requirement for norepinephrine is increasing progressively and her physicians are concerned that NO overproduction is resulting in desensitization to adrenergic agonists. At the same time, endogenous NO may have beneficial antimicrobial and respiratory-related functions. The physicians infuse hemoglobin 100 milligrams per kilogram IV in a composition containing 10 millimolar phosphate and 90 millimolar borate (to increase the NO reservoir as iron nitrosyl-Hb).

A patient with ischemia can be given a composition comprising hemoglobin at 100 milligrams per kilogram in 10 millimolar phosphate infused intravenously.

For a patient in sickle cell crisis, an infusion of 100 milligrams per kilogram IV hemoglobin can be infused in a composition containing 10 millimolar phosphate. Inorganic nitrite is added to the composition at a ratio of 1 per 100 hemoglobin molecules.

A patient with sickle cell disease presenting to the emergency room with chest syndrome is given inhaled nitric oxide at 40-80 parts per million with only slight improvement. She is begun on an infusion of 100 milligrams per kilogram hemoglobin in a composition containing 10 millimolar phosphate. After 3 units equivalents of hemoglobin are given, her symptoms begin to improve as measured by a decrease in oxygen requirement. She is subsequently given an infusion of 10 millimolar phosphate alone, in conjunction with inhaled nitric oxide.

Alternatively, upon presentation of chest syndrome, a patient with sickle cell disease can be given inhaled oxygen, to allow cooperative binding of NO to oxyhemoglobin. She can then be given inhaled NO and an infusion of 100 milligrams per kilogram hemoglobin in a composition containing a physiologically compatible buffer.

In another scenario, a patient with sickle cell disease presenting to the emergency room with chest syndrome is given 100 mg/kg hemoglobin along with inhaled nitric oxide at 40-80 parts per million, with no improvement. The hemoglobin is continued, but she is then given inhaled 70% oxygen instead, with no improvement. The hemoglobin is continued, but the 70% oxygen is discontinued, and a combination of the inhaled nitric oxide and 70% oxygen is given. With this therapy, the oxygen promotes the R structure of hemoglobin, allowing NO to bind. The patient improves, as measured by a decrease in oxygen requirement.

If the hemoglobin of a blood sample is tested for the cooperativity of NO binding to hemoglobin, and it is found that the most prevalent species of NO-modified hemoglobin is S-nitrosohemoglobin, and the predominant physiological effect of the composition is vasodilation, then further steps can be combined with the test steps to make a method of therapy for those in need of the effects of nitric oxide or its biological equivalent, wherein the steps further comprise administering to the patient added thiol, for example, administering to a human patient N-acetylcysteine IV at 50-100 mg/kg or PO at 600 mg 3 times per day.

As used herein, "NO" and "nitric oxide" include the biologically active forms of nitric oxide identified as being responsible for physiological functions such as smooth muscle cell relaxation, killing of bacteria and killing of bacteria by white blood cells, synaptic transmitter function, release of adrenaline from adrenal medulla, gut peristalsis, regulation of penile tone and inhibition of blood clotting. "NO" includes the free radical form as well as nitroxyl anion (NO⁻) and nitrosonium (NO⁺). It will be appreciated that NO exists in biological systems not only as nitric oxide gas, but also in various redox forms and as biologically active adducts of nitric oxide such as *S*-nitrosothiols, which can include S-nitrosoproteins, *S*-nitroso-amino acids and other *S*-nitrosothiols (Stamler, J.S. Cell 78:931-936 (1994)). Nitrosothiols (SNO), formed by nitrosylation ofthiols, can act as "carriers" of NO, in effect, extending the short physiological half-life of NO. Thus, carriers of NO such as SNO-hemoglobin can also be biologically active forms of nitric oxide.

A hemoglobin can be a naturally occurring protein of any animal or human, an active (having binding activity to NO and/or O₂, for example, or the activity of Equation 1 or Equation 2) variant thereof or an active fragment of a naturally occurring protein or active variant thereof. A variant hemoglobin typically differs in amino acid sequence from another reference hemoglobin. Generally, differences are limited so that the sequences of the reference polypeptide and the variant are closely similar overall and, in many regions, identical. A variant hemoglobin and a reference hemoglobin can differ in amino acid sequence by one or more amino acid substitutions, additions, deletions, truncations, fusions or any combination thereof. Variant hemoglobins include naturally occurring variants (e.g., allelic forms) and variants which are not known to occur naturally, such as fusion proteins. Non-naturally occurring variant hemoglobins can be produced using suitable methods, for example, by direct synthesis, mutagenesis (e.g., site directed mutagenesis, scanning mutagenesis) and other methods of recombinant DNA technology.

A hemoglobin to be administered in a method of therapy can be produced using suitable methods. For example, the hemoglobin can be obtained from cells in which it is produced (e.g., reticulocytes, recombinant cells) using conventional methods (e.g., homogenization, precipitation, differential centrifugation, chromatography, preparative electrophoresis). In one embodiment, the hemoglobin is isolated from the cells in which it is produced in nature. The term "isolated" as used herein indicates that the hemoglobin exists in a physical milieu which is distinct from that in which it occurs in nature. For example, an isolated hemoglobin can be substantially isolated with respect to the complex cellular milieu in which it naturally occurs, and can be purified essentially to homogeneity, for example as determined by analytical electrophoresis or chromatography (e.g., HPLC).

A hemoglobin can be administered to a mammal as part of a composition comprising an isolated hemoglobin and a pharmaceutically or physiologically acceptable carrier. Formulation will vary according to the route of administration selected (e.g.; solution, emulsion, capsule). Suitable physiological carriers can contain inert ingredients which do not interact with the hemoprotein. Standard pharmaceutical formulation techniques can be employed, such as those described in - Remington's Pharmaceutical Sciences, Mack Publishing Company, Easton, PA. Suitable physiological carriers for parenteral administration include, for example, sterile water, physiological saline, bacteriostatic saline (saline containing about 0.9% mg/ml benzyl alcohol), phosphate-buffered saline, Hank's solution, Ringer's-lactate and the like. Methods for encapsulating compositions (such as in a coating of hard gelatin or cyclodextran) are known in the art (Baker, et al., Controlled Release of Biological Active Agents, John Wiley and Sons, 1986). For inhalation, the agent can be solubilized and loaded into a suitable dispenser for administration (e.g., an atomizer, nebulizer or pressurized aerosol dispenser). In addition, a hemoglobin may be complexed into liposomes or micelles. A hemoglobin may be administered in combination with other drugs, or can be administered in combination with biologically compatible thiols, such as glutathione.

A blood substitute can be a biologically compatible liquid which performs one or more functions of naturally occurring blood found in a mammal, such as oxygen carrying and/or delivery, NO carrying and/or delivery, and the scavenging of free radicals. A blood substitute can also comprise one or more components of such a liquid which, when infused into a mammal, perform one or more functions of naturally occurring blood. Examples of blood substitutes include preparations of various forms of hemoglobin. Such preparations can also include other biologically active components, such as a low molecular weight thiol, nitrosothiol or NO donating agents, to allow transnitrosation. Low molecular weight thiols (i.e., relative to proteins and other biological macromolecules; e.g., see WO 98/34955 for further description that distinguishes low molecular weight thiols and nitrosothiols from those of high molecular weight) can include glutathione, cysteine, and *N-*acetylcysteine. *S*-nitrosothiols of low molecular weight can include *S-*nitrosocysteinylglycine, *S*-nitrosocysteine, *S*-nitrosohomocysteine, and *S-*nitrosothiols of a similar molecular weight range.

It is possible to achieve intracellular S-nitrosothiol, for example, by a process of removing whole blood from a patient's body (as a minimal method of isolating red blood cells, wherein the endogenous level of SNO-hemoglobin is about 0.3 µM), treating the red blood cells by addition of NO as described herein and then reintroducing the red blood cells into the same patient, thereby allowing the treatment of a number of types of diseases and medical disorders, such as those which are characterized by abnormal O₂ metabolism of tissues, oxygen-related toxicity, abnormal vascular tone, abnormal red blood cell adhesion, and/or abnormal O₂ delivery by red blood cells. Such diseases can include, but are not limited to, ischemic injury, hypertension, shock, angina, stroke, reperfusion injury, acute lung injury, sickle cell anemia, and blood borne infectious diseases such as schistosomiasis and malaria. The use of such red blood cells treated with NO also extends to blood substitute therapy and to the preservation of living organs, such as organs for transplantation. In some cases, it will be appropriate to treat a patient with NO-treated red blood cells originating from a different person. For sickle cell disease, the desired effect is to endow the red blood cell with vasodilator and antiplatelet activity, which should reverse the vasoocclusive crisis.

It has been observed that patients diagnosed with and patients at risk for cardiovascular disease present an increase in leukocytes, especially in neutrophils. Activated neutrophils produce toxic oxygen radicals. Exposure of red blood cells to oxygen radicals may produce oxidative damage in the membrane proteins. According to one theory, oxygen radicals contribute to the formation of atherosclerotic lesions. Total white blood cell count is elevated in the hypertensive population and in the population having suffered a myocardial infarction, mostly due to higher neutrophils. Other differences include increases in mean cell volume and decreases in G6PD activity. Low G6PD activity is often used as an indicator of erythrocyte senescence, which may be due, in part, to oxidative damage to the cells. Membrane-bound hemoglobin was found to be significantly higher in red blood cells from patients at risk for cardiovascular disease than in red blood cells from healthy control patients at much lower risk. See Santos-Silva, A., et al., Atherosclerosis 116:199-209, 1995.

A method included in the disclosure is a therapy for inflammatory conditions, for example, arthritis, asthma, cerebritis, bronchitis, vasculitis, etc. The method is to disrupt NO export from red blood cells by administering to the patient an inhibitor of AE1 anion transport function, or an agent that interferes with the interaction between hemoglobin and AE1. The anion exchange protein (AE1; also band 3 protein) consists of 911 amino acid residues and is composed of two domains, a 52-kD membrane spanning domain, which mediates the efflux of HCO₃⁻ from the cell in exchange for Cl⁻ and an amino terminal 43-kD cytoplasmic domain. Three classes of AE1 inhibitors have been proposed. See, for example, Falke, J. and S.I. Chan, Biochemistry 25:7895-7898, 1986). The amino terminal 43-kD cytoplasmic domain has a site for the association of several proteins, including hemoglobin, as observed through the binding ofhemochromes (See, for example, Waugh, S.M. et al., Biochemistry 26:1777, 1987).

A fragment of the cytoplasmic domain of AE1 comprising the binding site of AE1 for hemoglobin or a mimetic of this binding site can be used in a method of therapy to regulate the release of nitric oxide or its biological equivalent from the erythrocyte, by disrupting the interaction between hemoglobin and AE1. For example, a peptide consisting of the 11 N-terminal amino acid residues of AE 1 was found to bind to hemoglobin (Walder, J.A. et al., J. Biol. Chem. 259:10238-10246, 1984).

Another embodiment of the disclosure arising out of the studies described herein is a method for facilitating the release of NO from nitrosated hemoglobin, for example, SNO-hemoglobin, the method comprising administering to a mammal (*e.g.*, human patient) a composition (*e.g.*, a blood substitute) comprising SNO-hemoglobin and which may also comprise other forms of purified hemoglobin, and an agent that facilitates the release of NO from SNO-hemoglobin, thereby causing the physiological effects mediated by NO, such as smooth muscle relaxation, vasodilation and the resultant decrease in blood pressure, inhibition of platelet activation and platelet aggregation, etc.

The agent can be, in one instance, a peptide having an amino acid sequence exactly matching the amino acid sequence of an N-terminal fragment of AE1 protein, wherein the peptide has the amino acid sequence of the first 11 amino acid residues of AE1 protein, MEELQDDYEDE (SEQ ID NO:1). In another instance, the agent can be a peptide with one or more amino acid substitutions compared to SEQ ID NO:1, where conservative substitutions of one amino acid residue for another with similar properties (*e*.*g*., hydrophobic, aromatic, polar, basic, acidic, small) are preferred. In other instances, the agent can be a peptide with one or more additions or deletions of amino acid residues. Substitutions, additions and deletions can occur in various combinations. In further instances, the agent can be a modified peptide or a non-peptide mimetic of the peptide of SEQ ID NO: 1. Where it is desired that an agent not having SEQ ID NO:1 have equivalent or similar effects on hemoglobin, the binding properties of the agent to isolated hemoglobin and the effect of the agent on the oxygen binding curve for hemoglobin can be assessed by methods as described by Walder, J.A. et al., J. Biol. Chem. 259(16):10238-10246, 1984, or methods similar to those as known to persons of skill in the art. By the methods described in Walder *et al.,* agents that facilitate the release of NO from hemoglobin, with the resultant physiological effects of NO release, can be identified.

In other instances, the agent that facilitates the release of NO from SNO-hemoglobin by promoting the T structure ofhemoglboin can be a peptide (which may be of such a length to also be termed a polypeptide) having an amino acid sequence matching that of an N-terminal fragment of AE1 which is at least 201 amino acid residues long starting from amino acid residue 1 of SEQ ID NO:2 (SEQ ID NO:3 consisting of amino acid residues 1-201 of SEQ ID NO:2), or at least 317 amino acid residues long starting from amino acid residue 1 of SEQ ID NO:2 (SEQ ID NO:4 consisting of amino acid residues 1-317 of SEQ ID NO:2).

As in the case of SEQ ID NO: 1, the agent can be a peptide with one or more amino acid substitutions compared to SEQ ID NO:3 or SEQ ID NO:4, where conservative substitutions of one amino acid residue for another with similar properties (*e*.*g*., hydrophobic, aromatic, polar, basic, acidic, small) are preferred. In other instances, the agent can be a peptide with one or more additions or deletions of amino acid residues. Substitutions, additions and deletions can occur in various combinations, compared with the amino acid sequences of SEQ ID NO: 3 and SEQ ID NO:4. In further instances, the agent can be a modified peptide or a non-peptide mimetic of SEQ ID NO:3 or SEQ ID NO:4.

The peptide or polypeptide can be produced by proteolytic digestion of an isolated AE1 protein, or can be synthetically produced. The peptide, polypeptide, modified peptide or non-peptide agent to be used as an agent for NO release from hemoglobin can be a fragment of a human or other AE1 protein, or can be modeled after the structure of a portion of human or other AE1 protein.

An agent that facilitates the release of NO from SNO-hemoglobin can also be one that can be administered to a mammal to act on the proteins within red blood cells. A peptide or polypeptide agent of this type can comprise the same amino acid sequence as those peptides or polypeptides administered to act on isolated hemoglobin, and can also comprise a second portion, covalently bound as a fusion peptide or polypeptide, wherein the second portion is a protein or peptide that by itself enters cells through the cytoplasmic membrane and can facilitate the entry of covalently bound peptides or polypeptides as fusion polypeptides or peptides. For example, see Prochiantz, A. et al., Curr. Opin. Cell Biol. 12(4):400-406, 2000, for some "vector" peptides or proteins.

Hemoglobin can be regulated by those factors that affect the R/T transition and the spin state. In addition to the physiological factors that vary with site in the circulation (pH, pCO₂, and pO₂), other regulators of R/T transition can be used to mediate hemoglobin's role in carrying and releasing nitric oxide.

Inhibitors or enhancers of carbonic anhydrase II (found in erythrocyte cell membranes and elsewhere, for example, hepatocytes) can be administered to a mammal in a method of therapy to regulate blood pressure and other NO-mediated physiological effects. Inhibitors of carbonic anhydrase II include, for instance, (4*S-trans*)-4-(ethylamine)-5,6-dihydro-6-methyl-4*H*-thieno[2,3-6]thiopyran-2-sulfonamide 7,7-dioxide monohydrochloride (also called dorzolamide hydrochloride or Trusopt™ by Merck Pharmaceuticals), 4,5-dichloro-1,3-benzendisulfonamide, acetazoamide (Lipsen, B. and R.M. Effros, J. Appl. Phsyiol. 65(6):2736-2743, 1988), methozolamide, MK-927, L-662,583 (M.F. Sugrue et al., Br. J. Pharmacol. 99:59-64, 1990), and L-693,612 (Wong, B.K. et al., Pharm Res. 11(3):438-441, 1994). To decrease the release of nitric oxide biological activity from red blood cells in a mammal, an effective amount of a composition comprising an inhibitor of carbonic anhydrase II activity can be administered to the mammal.

As red blood cells are stored, they lose NO (to a level at least as low as 0.3 µM, and possibly as low as 50 nM) as they are away from their source of NO - the endothelial cells of blood vessels. Adding NO to stored red blood cells can be done to restore the level of NO to its physiological level (to greater than 50 nM which may be found in stored RBCs) or to add extra NO for the physiological effects of NO upon administration of the red blood cells. A further benefit of adding NO to isolated red blood cells being stored for later administration to a patient in need of blood is that NO is protective against the effects of oxidation, senescence, and lipid peroxidation that can occur during storage.

Oxygenated red blood cells of a human or other mammal can be contacted with a liquid solution comprising NO dissolved gas, wherein the pH is in the range of about 7.4 to 9, the temperature is about 25°C to 37°C, and the buffer in which the NO gas is dissolved is chosen to be physiologically compatible with maintaining the osmotic pressure of the cells and with maintaining the R structure of hemoglobin. For example, the buffer can be 10 mM phosphate buffer, or Krebs buffer.

Deoxygenated red blood cells of a human or other mammal can be contacted with a liquid in which NO gas has been dissolved. Appropriate amounts of the composition comprising the NO gas can be added to the red blood cells until a NO:heme ratio of approximately 1:50 to 1:1000 is reached. The composition comprising the dissolved NO gas can be, for example, 10 mM phosphate buffer, Krebs buffer, or some other physiologically compatible buffer that allows for the formation of SNO-hemoglobin as measured, where a physiologically compatible buffer allows for the correct folding and function of the proteins of the red blood cell, and the correct osmotic balance. The temperature can be in the range of about 25°C to about 37°C. After the addition of NO, the red blood cells can be oxygenated before administration to the human or other mammal.

It is a further method of the disclosure to provide a method to restore red blood cells in the circulation of a mammal to normal levels. This method finds application in the treatment of injuries or anemias, such as sickle cell anemia and thalassemias. The method comprises administering to the patient a composition comprising red blood cells which have been treated to restore the endogenous level of NO found in normal red blood cells in the body (approximately 0.3 µM), or comprising red blood cells which have been treated to comprise NO at a level which is higher than that naturally found in freshly drawn blood.

The compounds and therapeutic preparations of this disclosure to be used in medical treatment are intended to be used in therapeutically effective amounts, in suitable compositions, which can be determined by one of skill in the art. Modes of administration are those known in the art which are most suitable to the affected site or system of the medical disorder. Intravenous infusion is a preferred mode of administration of various forms of hemoglobin to be used as a blood substitute. Suitable compositions can include carriers, stabilizers or inert ingredients known to those of skill in the art, along with biologically active component(s).

The term "therapeutically effective amount," refers to the amount of blood substitute, isolated erythrocytes, drug, modified Hb and/or form of NO or NO donor, etc., which is effective to achieve its intended purpose. While individual needs vary, determination of optimal ranges for effective amounts of each therapeutic agent to be administered is within the skill of one in the art. Research animals such as dogs, baboons or rats can be used to determine dosages. Generally, dosages required to provide effective amounts of the composition or preparation, and which can be adjusted by one of ordinary skill in the art, will vary, depending on the age, health, physical condition, sex, weight, extent of disease of the recipient, frequency of treatment and the nature and scope of the desired effect. Dosages for a particular patient can be determined by one of ordinary skill in the art using conventional considerations, (*e.g*. by means of an appropriate, conventional pharmacological protocol). For example, dose response experiments for determining an appropriate dose of a heme-based blood substitute can be performed to determine dosages necessary to produce a physiological concentration of approximately 1 nM to 100 µM heme. Red blood cells loaded with NO have been demonstrated to lower blood pressure (see Example 8). Dose-dependent blood pressure lowering can be achieved by NO-loaded RBCs containing up to 1:50 SNO:hemoglboin. Suitable pharmaceutical carriers or vehicles can be combined with active ingredients employed in a therapeutic composition, if necessary.

The findings described in Examples 6, 7 and 8 indicate that the interaction of Hb with AE1 must be incorporated in the scheme that organizes the reactions governing the fate of NO within the RBC. Figure 10 is a diagram representing the following scheme. NO reaction pathways depend on whether Hb is cytosolic or membrane-associated. NO entering the cytosol of the RBC will participate in the R/T-regulated equilibrium between SNO-Hb and iron nitrosyl Hb that conserves NO on the one hand, and between SNO-Hb and *S*-nitrosoglutathione that generates bioactivity on the other (Gow, A.J., et al., Nature, 391:169-173, 1998; McMahon, T.J., et al., J. Biol. Chem., 275:16738-16745, 2000). However, endothelial-derived NO will first encounter the zone of membrane-associated Hb with which it will preferentially interact. The site of Hb binding within the cytoplasmic domain of AE1 has been localized to the polyanionic N-terminus, and analysis of co-crystals of Hb and an AE1 N-terminal peptide has shown that this stretch of acidic residues inserts into the 2,3-diphosphoglycerate-binding pocket formed between β-globin subunits of tetrameric Hb (Walder, J.A., et al., J. Biol. Chem., 259:10238-10246, 1984). Consistent with this binding mechanism, AE1 binds with higher affinity to deoxyHb than to oxyHb (in which the β-cleft is occluded) (Walder, J.A., et al., J. Biol. Chem., 259:10238-10246, 1984: Chetrite, G., et al., J. Mol. Biol., 185:639-644, 1985), and consistent with the requirements of thermodynamic linkage (McMahon, T.J., et al., J. Biol. Chem., 275:16738-16745, 2000), the oxygen affinity of Hb is reduced in the presence of isolated AE1 cytoplasmic domain (Walder, J.A., et al., J. Biol. Chem., 259:10238-10246, 1984). SNO-Hb predictably exhibits similar allosteric responsivity to AE1 (not shown). Thus, AE1 is both an allosteric regulator that promotes Hb T-state and an acceptor of NO groups transferred by SNO-Hb upon R to T transition. Therefore, *S*-nitrosvlation of AE1 is a preferred outcome of SNO-Hb deoxygenation at the membrane-cytosol interface (Figure 10). AE1 is present at about 10⁶ copies per RBC (Pawloski. J.R., et al., Circulation, 97:263-267, 1998) and thus at about tenfold excess over SNO-Hb in arterial RBCs (Jia, L., et al., Nature, 380:221-226, 1996; Stamler, J.S., et al., Science, 276:2034-2037, 1997), sufficient for the proposition that transfer to AE I constitutes a major route of NO trafficking as the RBC transits the physiological pO₂ gradient (Stamler, J.S., et al., Science, 276:2034-2037, 1997).

The functional compartmentalization of the RBC suggested by our results has an additional implication for the physiological role of the RBC in regulating NO bioavailability. It has been shown that RBCs possess an unspecified intrinsic barrier to consumption of extracellular NO of sufficient strength that NO is consumed by RBCs as much as 1000 times slower than by equivalent concentrations of free Hb (Liu, X., et al., J. Biol. Chem., 273:18709-18713, 1998; Vaughn, M.W., et al., J. Biol. Chem., 275:2342-2348, 2000). This functional barrier may represent the juxta-membrane compartment in which transfer and export of Hb-derived NO are concentrated. That is, by impeding access to the cytosol, membrane-associated Hb may substantially decrease the effective concentration of Hb available to exogenous NO. Moreover, T-state Hb (bound preferentially by AE1) is intrinsically less avid for NO because T-structure molecules lose cooperative NO binding (Gow, A.J., et al., Proc. Natl. Acad. Sci., 96:9027-9032, 1999). Indeed, our bioassay data pointing to reduced consumption by RBCs of extracellular NO at low vs. high pO₂ would have a mechanistic basis in the increased concentration at the membrane-cytosol interface of (T-state) Hb, which would result from the increased affinity of deoxyHb for AE1 (Figure 10). This concerted action of RBCs to decrease scavenging of NO at low pO₂ would help fulfill the local metabolic requirement for coordinate delivery of O₂ and vasodilatory NO bioactivity.

Although the findings of Examples 6, 7, and 8 indicate that DIDS-induced inhibition of export of NO bioactivity can be accounted for by reduced transfer of NO groups from SNO-Hb to cysteine thiols within the cytoplasmic domain of AE1, a role for the anion exchange function of AE1 in transmembrane transport of NO bioactivity should also be considered. The anion selectivity of AE1 is not strict: erythrocyte AE1 has been shown to transport NO₂⁻, NO₃⁻ and OONO- (Galanter, W.L., et al., Biochim. Biophys. Acta, 1079:146-151, 1991; Shingles, R., et al., J. Bionerg. Biomembr., 29:611-619, 1997; Soszynski, M., et al., Biochem. Mol. Biol. Int., 43:319-325, 1997), and transport of NO⁻ would be expected. Thus, while it has been demonstrated that RBCs can release *S*-nitrosylated small molecular weight thiols (Jia, L., et al., Nature, 380:221-226, 1996), it is an appealing possibility that NO bioequivalents transit the RBC membrane at least in part by means of AE1-mediated transport of an NO congener or NO*ₓ* derived in the immediate juxta-membrane locale from AE1-liganded (S)NO.

In sum, our results provide a new perspective which indicates that translocation of NO bioactivity across membranes--studied here for the first time--is based upon segregation at the membrane of reaction pathways for transfer of NO equivalents, and that this compartmentalization in the RBC derives from specific protein-protein interactions of Hb. Our findings provide the first experimental demonstration of protein-protein transfer of NO groups in signal transduction, and may signify a widespread and critical role for transnitrosylation in transport and targeting of NO bioactivity (Stamler, J.S., et al., Neuron, 18:691-696, 1997). Finally, our results may provide new insight on the pathogenesis of the thrombotic diatheses, ischemic syndromes and hypertensive states that are associated with hemoglobinopathies (*e.g.* thalassemias, sickle cell disease), with RBC membrane defects (*e.g.* band 3 deficiency, malaria, stomatocyosis, paroxysmal nocturnal hemoglobinuria) and with altered hematocrit (polycythemia, anemias), as well as on basic but poorly understood changes in RBC rheology and function (*e*.*g*. stored blood, chemotherapy and pharmacotherapy).

It has recently been demonstrated that, under physiological conditions, the interplay of NO and mammalian Hb is governed primarily by a dynamic oxygen-regulated equilibrium between two species that differ according to whether the NO group is liganded to heme iron or to a highly-conserved cysteine thiol within the β-globin subunit (cysβ93) (Gow, A.J., et al., Nature, 391:169-173, 1998; Gow, A.J., et al., Proc. Natl. Acad. Sci. USA. 96:9027-9032, 1999; McMahon, T.J., et al., J. Biol. Chem., 275:16738-16745, 2000). NO is bound preferentially by heme when Hb is in the deoxygenated T (tense) structure (to yield iron nitrosyl Hb: HbFe(II)NO) and by cysβ93 in the oxygenated R (relaxed) structure (to yield *S*-nitroso Hb: SNO-HbFe(II)O₂) (Gow, A.J., et al., Nature, 391:169-173, 1998; McMahon, T.J., et al., J. Biol. Chem., 275:16738-16745, 2000). The allosteric transition from R to T state effects transfer of NO groups from cysβ93 to heme, which acts to conserve NO (Jia, L., et al., Nature, 380:221-226, 1996; McMahon, T.J., et al., J. Biol. Chem., 275:16738-16745, 2000), and also to a thiol acceptor such as glutathione (GSH), which allows for intermolecular transfer of a bioactive NO congener (Gow, A.J., et al., Nature, 391:169-173, 1998; McMahon, T.J., et al., J. Biol. Chem., 275:16738-16745,2000).

These findings support a model under which the physiological O₂ gradient is transduced by Hb into a coordinate release by RBCs of O₂ and SNO-derived vasoactivity, to optimize oxygen delivery in the arterial periphery (Jia, L., et al., Nature, 380:221-226, 1996; Stamler, J.S., et al., Science, 276:2034-2037, 1997). However, although the bioactivity of cell-free SNO-Hb has been established (McMahon, T.J., et al., J. Biol. Chem., 275:16738-16745, 2000; McMahon, T.J., et al., Meth. Enzymol., 301:99-114, 1999), and RBCs can release NO-related bioactivity (Jia, L., et al., Nature, 380:221-226, 1996; Stamler, J.S., et al., Science, 276:2034-2037, 1997; Pawloski, J.R., et al., Circulation, 97:263-267), the mechanism that operates within RBCs to couple the R to T transition of SNO-Hb to export of vasodilatory activity remained unknown prior to the studies described herein.

SNO-Hb is present in arterial blood at a concentration of about 0.3 µM (Jia, L., et al., Nature, 380:221-226, 1996; Stamler, J.S., et al., Science, 276:2034-2037, 1997), and only 0.1-1% of thiol-liganded NO will actually be released by R to T transition during a single arterio-venous transit, as Hb effectively conserves the remaining NO (Gow, A.J., et al., Nature, 391:169-173, 1998; McMahon, T.J., et al., J. Biol. Chem., 275:16738-16745, 2000). This dynamic serves clamant physiological needs, since NO release rates would otherwise overwhelm NO production by NO synthase, and also excessively lower blood pressure (McMahon, T.J., et al., J. Biol. Chem., 275:16738-16745, 2000). NO equivalents are thus, in principle, available for export by RBCs at levels commensurate with the low nanomolar flux necessary and sufficient for regulation of blood flow (Stamler, J.S., et al., Science, 276:2034-2037, 1997; McMahon, T.J., et al., J. Biol. Chem., 275:16738-16745, 2000). However, since both Hb and GSH in the RBC cytosol are in vast excess over SNO-Hb/GSNO, reaction pathways that would prevent entry of NO equivalents into a bioactive and exportable pool would be highly favored on the basis of simple mass-action constraints. Evidently, there must exist within the RBC the means to discriminate between nitrosylated and unliganded molecules and/or to sequester SNO bioactivity.

### EXEMPLIFICATION

### Reaction Product Analysis

To investigate the reaction of NO with oxyHb, we begin by adopting the conventional viewpoint that: NO consumption involves a competition between the oxidation reaction (Eq. 1) and the adduct-forming addition reaction (Eq. 2); and that the specific rate constants for these reactions, namely kₒₓ and k_{add}. are independent of the degree of oxygen saturation (Y) of the hemes.

[1] Fe(II)O₂ + NO → Fe(III) + NO₃⁻

[2] Fe(II) + NO → Fe(II)NO

These two assumptions define a perspective of the NO reaction that we refer to as the "simple competition model." Our analysis of the reaction products, as described in this section, enables us to test the adequacy of this model for describing the chemistry and to recognize and interpret deviations from the behavior implied by it.

In our experiments, NO is introduced as a limiting reagent in an amount substantially smaller than the total amount of oxy- and deoxyhemes. On completion of the reaction, the following relation can be shown to exist among the products:

[Fe(II)NO]/[Fe(III)] = (k_{add}/kₒₓ)([Fe(II)]₀/[Fe(II)O₂]₀) [3]

in which [Fe(II)]ₒ and [Fe(II)O₂]_{0,} are respectively the initial concentrations of deoxy- and oxyheme. The simple form of Eq. 3 takes advantage of the fact that, independent of Y, at least one of the reactions proceeds under pseudo-first order conditions, and that
kₒₓ ≈ k_{add}. The mass balance constraint [Fe(II)NO] + [Fe(III)] = [NO]_{0.} enables us to express the product concentrations relative to the initial concentration of NO, namely [NO]₀, as:

[4] [Fe(II)NO]/[NO]₀ = 1 - Y/[1 - Y + κY]

in which κ is kₒₓ/k_{add}, and

[5] [Fe(III)]/[NO]₀ = 1 - [Fe(II)NO]/[NO]₀

Eqs. 4 and 5 provide the key relationships by which we test the simple competition model -- specifically, Eq. 4 indicates that the fractional yield of nitrosylhemoglobin (nitrosylHb) as a function of Y assumes the form of an arc, ranging from 100% yield at Y = 0 to 0% yield at Y = 100%. The degree of curvature of the arc is determined by κ; it is a straight line for κ = 1, but is bowed to one or the other side of this diagonal line as κ is alternatively increased or decreased. For no value of κ does the curve cross the diagonal. It is also worth noting that the derivative of the curve is given by:

[6] d([Fe(II)NO]/[NO]₀)/dY = -κ/[1 - Y + κY²]

hence as Y → 0, the tangential slope is -kₒₓ/k_{add}, and Y → 1, it is -k_{add}/kₒₓ. These properties are useful for recognizing possible Y dependences of κ that are inconsistent with the simple model. Similarly, Eq. 5 provides a test for the presence of additional reactions, beyond oxidation (Eq. 1) and addition (Eq. 2): if additional reactions are significant, then [Fe(III)] and [Fe(II)NO] will not account for the total NO ([NO]₀) consumed in the reaction, whence [Fe(III0] /[NO]₀ + [Fe(II)NO]/[NO]₀ < 1.

### NO Treatment of Hb

HbA₀ was obtained from Apex Bioscience (Research Triangle Park, NC). Buffer exchange was achieved by dialysis. Deoxygenation was performed by gas exchange with argon in a tonometer. NO was added from a stock solution prepared under ultrapure helium and purified across alkali and cold traps. Stock solutions of NO were prepared in phosphate buffered saline containing 100 µM DTPA, pH 7.4. NO injections were made via a gas tight Hamilton syringe with Teflon seal. The concentration of NO in stock solutions was assayed by electrode and by a Sievers 280 NO analyzer (Boulder CO).

### Titration of Normoxic Hb with NO

Air-oxygenated Hb was titrated with 0.22 µM NO. Samples were analyzed immediately after NO addition by UV-visible spectrophotometry. Time between additions varied from 3 to 5 minutes.

### Measurements of S-Nitroso-, Iron Nitrosyl- and Met-Hb

Nitroso/nitrosyl derivatives of Hb were measured using a photolysis-chemiluminescence technique [6-fold excess HgCl₂ over protein was added to displace *S*-nitrosothiol (SNO) (Stamler, J.S., et al., Science, 276:2034-2037, 1997)]. Samples were kept on ice for a period of 5 minutes to 2 hours before analyses. MetHb was monitored by UV-visible spectroscopy as the difference absorption above the linear baseline (600-700 nm), and EPR (below).

### EPR analysis

EPR spectroscopy was carried out with samples in 4-mm i.d. fused silica tubes, at 76° K, on a Varian E-9 spectrometer. UV-visible spectra were taken after NO addition. The sample was then placed in a deoxygenated EPR tube and plunged into liquid N₂. EPR spectra of nitrosylHb or dinitrosyl iron complexes (DNICs) were recorded in a single 4-min. scan over 400 G on a Varian E-9 spectrometer operating at 9.274 GHz, with 10-mW microwave power, 10-20 G amplitude of field modulation at 100 kHz, and time constant of 0.250 sec. Spectra of high-spin metHb were recorded with a scan of 1000 G, 20 G modulation amplitude, time constant of 0.128 sec, under otherwise identical conditions. NitrosylHb was measured by double integration of EPR spectra and by comparison to EPR spectra of Hb(NO)₄ standardized with UV-visible spectroscopy. The reproducibility of nitrosylHb measurements was estimated to be ±6% by repeated trials.

### Measurement of Oxygen Saturation

Oxygen saturation of Hb was verified by UV-visible spectroscopy using 1-mm anaerobic cuvette.

### Extinction Coefficients

The extinction coefficient spectra of metHb, deoxyHb, iron nitrosylHb and oxyHb were generated from pure solutions of each species. HbA was diluted into PBS (pH 7.4) to a known final heme concentration [as calculated by the pyridine-hemochromagen method (Antonini, E., et al., p. 13 in Frontiers in Biology, Neuberger, A. and Tatum, E.L. eds., North-Holland Publishing, Co., Amsterdam, London, 1971)]. MetHb was synthesized by adding excess K₃FeCN₆. DeoxyHb was measured after the addition of dithionite, and nitrosyl- and oxyHb were measured following saturation with each ligand.

### Modeling of UV-Visible Difference Spectra

Difference spectra were obtained by subtracting the UV-visible spectrum of a given sample before the addition of NO from those after. The simple competition model discussed above, predicts that such difference spectra could be approximated by a linear combination of two standard difference spectra: an oxyHb minus metHb spectrum, which gauges the progress of the NO oxidation reaction, and a deoxyHb minus iron nitrosyHb, which gauges the NO addition reaction; the sum of the combining coefficients is fixed by the mass balance ([NO]₀). Standard difference spectra were obtained from UV-visibie spectra of authentic samples of metHb, oxyHb, nitrosylHb, and deoxyHb. We determined combining coefficients by a least-squares fitting procedure. Inasmuch as the deoxy- and/or oxyheme concentrations can decline during the competition, an additional component (deoxyHb minus oxyHb) could be expected.

### Reactions ofNO/SNO with RBCs and IOVs (Figure 7)

RBCs derived from fresh venous blood and suspended in oxygen-purged phosphate-buffered saline (PBS) were exposed in gas-tight vials to NO, added as aliquots of oxygen-purged NO-saturated PBS to yield the specified NO:heme ratios based on spectrophotometric assessment of heme content. Exposure to NO was for 5 mins followed by rinses in PBS at 21% O₂. RBCs were lysed in 10 mM MOPS, 0.1 mM DTPA, pH 7, followed by centrifugation at 20,000 x g for 10 mins to produce membrane and cytosolic fractions, which were solubilized in 1% TX-100. Quantification of NO groups was by photolysis/chemiluminescence (McMahon, T.J. & Stamler, J.S., Meth. Enzymol. 301:99-114,1999). SNO content was measured as NO removed by treating sample aliquots for mins with HgCl₂ at 5-fold molar excess over estimated thiol content (Gow, A.J. & Stamler, J.S., Nature 391:169-173,1998; Gow, A.J. et al., Proc. Natl. Acad. Sci. USA 96:9027-9032,1999; McMahon, T.J. & Stamler, J.S., Meth. Enzymol. 301:99-114,1999). IOVs were prepared from outdated RBCs following Kondo (Kondo, T., Meth. Enzymol. 171:217-225, 1989) and stripped of peripheral membrane-bound proteins by washing for 60 mins in 0.5 M NaCl/100 mM Tris at pH 8. Chymotrypsin treatment of IOVs was for 15 mins at pH 7 and 37°C with 5 U enzyme/mg IOV protein. For transnitrosylation, IOVs were incubated with SNO-Hb (50 nmoles SNO-Hb/mg IOV protein, about 10-fold excess over AE1) for 15 mins at pH 7 and 37°C. SNO-Hb was prepared from free or immobilized HbA (Apex) by selective *S*-nitrosylation of cysβ93 with *S*-nitroso-cysteine (Jia, L. et al., Nature 380:221-226, 1996; Stamler, J.S. et al., Science 276:2034-2037, 1997; McMahon, T.J. & Stamler, J.S., Meth. Enzymol. 301:99-114, 1999). Hb was immobilized on cyanogen bromide-activated Sepharose 4b.

### Immunoprecipitation from extracts of RBCs and IOVs, and DIDS-modification (Figure 8)

Immunoprecipitation was carried out by incubation of TX-100 or NP-40 extracts with antibodies followed by protein G-Sepharose. We employed two monoclonal anti-AE1 antibodies (Sigma, clone BIII-136, and kindly provided by M. Telen) that recognized epitopes in the cytoplasmic domain and that produced indistinguishable results. Immune complexes were eluted with 10 mM glycine at pH 3.0, and eluates were adjusted immediately to pH 7.4 followed by photolysis/chemiluminescence. Efficiency of immunoprecipitation was confirmed by Western blotting. Binding of SNO-Hb to IOVs was assessed in 10 mM MOPS buffer, pH 7, at room temperature and 21 % O₂. Incubation was for 15 mins before IOVs were solubilized in I % TX-100, and heme content was assessed spectrophotometrically. When employed, RBCs were treated for 60 mins with DIDS (0.1 mM; calculated 10-fold molar excess over AE1), then washed thoroughly to remove unreacted DIDS. RBCs were then exposed to NO (as above) or used to prepare DIDS-modified IOVs (as above).

### Bioassay of NO activity (Figure 9)

Rabbit aortic rings were suspended in Krebs-bicarbonate buffer at 37°C, bubbled continuously with either 95% O₂/5% CO₂ or 95% argon/5% CO₂ (measured O₂< 1%) (Jia, L. et al., Nature 380:221-226, 1996; Stamler, J.S. et al., Science 276:2034-2037, 1997). Resting tension was maintained at a standard 2 gm with phenylepherine. NO-treated and control RBCs were washed and resuspended in PBS at 50% hematocrit, then added to individual 25 ml baths as 0.2 mL aliquots to yield a bath hematocrit of 0.4%.

### Example 1. Production of iron nitrosylHb by addition of NO to variously oxygenated Hb (see Figures 1A-1D)

(A) EPR spectra of iron-nitrosyl Hb derivatives formed by incubation of 19 µM NO with 393 µM Hb at various degrees of oxygen saturation in 10 mM phosphate buffer, pH 7.4. The oxygen saturations for the largest through smallest EPR signals are 5.5, 32, 50 and 69%, respectively. Spectra show predominantly 6 coordinate α and β nitrosyl hemes, as typically observed for Hb in R state. (B) EPR spectra of iron-nitrosyl Hb derivatives formed by incubation of 55 µM NO with 380 µM Hb at various degrees of oxygen saturation in 100 mM phosphate, pH 7.4. The oxygen saturations for the largest through smallest EPR signals are 1, 15, 41, 60 and 80%, respectively. Spectra show a significant component of five coordinate α nitrosyl hemes (triplet structure), associated with Hb in T state. (C) Trials conducted with Hb in 10 mM phosphate, pH 7.4. The symbols are experimental results and the solid lines represent a best fit to the functional form for cooperative NO binding. Open diamonds, 393 µM Hb incubated with 19 µM NO; open circles, 350 µM Hb incubated with 15 µM NO plus 0.05% borate (added to bring the buffer concentration to 100 mM as in D); open squares, 365 µM Hb incubated with 15 µM NO and 1, 190 units/ml SOD. (D) Trials conducted with Hb in 100 µM phosphate, pH 7.4. The symbols are experimental results and the lines represent a best fit to the functional form for simple competition between oxidation and NO addition reactions (Eq. 4). Filled circles, 380 µM Hb incubated with 55 µM NO; filled squares 375 µM Hb incubated with 7 µM NO. Application of the simple competition function to data of C or the cooperativity function to data of D gives an order of magnitude increase in χ².

### Example 2. Production of metHb by reaction of NO is disfavored with increasing oxygen saturation (see Figure 2)

The samples used in Figures 1A-1D were assayed for metHb production by UV-visible difference spectroscopy. The data are normalized to added [NO]. As in Figures 1A-1D: open diamonds, 10 mM phosphate; open circles, 10 mM phosphate plus borate; open squares, 10 mM phosphate plus SOD; filled circles and filled squares, 100 mM phosphate. The dotted (10 mM phosphate) and dashed (100 mM phosphate) lines are calculated by using Eq. 5 and Fe(II)NO yields in Figures 1C and 1D, respectively. Data show metHb to be disfavored in low phosphate, particularly at high oxygen saturation. Deviations of the data points below the curves suggest the presence of additional reactions for NO. Systematic deviations are most pronounced in low phosphate at high oxygen saturation -- i.e., under physiological conditions.

### Example 3. NO addition under normoxic conditions (≈99% O₂ saturation) produces nitrosylated Hb (see Figures 3A -3D)

(A) Nitrosyl content of oxyHb (10 mM phosphate 100 µM DTPA, pH 7.4) after exposure to 1.2 µM NO, as measured by photolysis-chemiluminescence (Stamler, J.S., et al., Science, 276:2034-2037, 1997). Nitrosyl yield increases as a function of Hb concentration (P < 0.05). Solid symbols, absolute yield of NO bound to Hb (FeNO plus SNO); open symbols, percentage of NO added. Data shown are the average of 7 to 19 experiments ± SE. (B) Standard difference spectra of metHb (solid line), deoxyHb (dotted line), and iron nitrosylHb (dashed line) vs. oxyHb. (C) Difference spectra generated from the exposure of NO to normoxic (≈99% oxygen saturation) Hb. NO was added (in 10 aliquots totaling 2.2 µM) to 33 µM Hb in 100 mM phosphate (solid line) or 10 mM phosphate (dotted line) or 10 mM phosphate plus 0.05% borate (dashed line). Notably, the spectrum in 100 mM phosphate shows the formation of metHb (*e.g.,* peak at 630 nm, see B for comparison); the spectrum in 10 mM phosphate shows formation of iron nitrosyl Hb and some metHb [*e*.*g*., peak at 595 nm (nitrosyl) and small peak at 630 nm (met), see B for comparison]; and the spectrum in 10 mM phosphate plus borate shows predominantly iron nitrosylHb (*e*.*g*., peak at 595 nm, see B for comparison). (D) Calculated fits for difference spectra shown in C, demonstrating simple (non-cooperative) competition between NO binding and oxidation reactions in high phosphate (solid line, 95% metHb) and cooperative binding in low phosphate (dotted line, 54% iron nitrosylHb; only 50% of the added NO accounted for) and low phosphate plus borate (dashed line; 85% iron nitrosylHb). Specifically, spectra in C were fitted, by a least-squares process, to either the simple competition model or the cooperativity model without a mass balance constraint. Cooperativity is present if k_{add} increases above that reported in the literature, as a function of oxygen tension.

### Example 4. S-nitrosoHb and iron nitrosylHb formed under various physiological, air-oxygenated conditions (see Figure 4)

(A) SOD increases the yield of NO bound to Hb. The experiments in Figure 3A were repeated in the absence (solid line; 1.2 µM NO) or presence (dashed line; 1.5 µM NO) of 1, 190 units/ml of SOD, which enhances the yield of nitrosyl species to approximately 100% of the NO added. Similar nitrosyl yields were obtained by using stroma-free Hb (25 µM), which is enriched in endogenous SOD (open circle). Data shown are the average of five to nine experiments ± SE. (B) EPR spectrum of a DNIC formed by exposure of oxyHb (=99% sat; 3.93 mM) to NO (36 µM). (C) *S*-nitrosoHb and iron nitrosylHb formed by exposure of oxyHb (≈99% sat., 48 µM) to NO (1.2 µM). SNO (hatched bar) and FeNO (solid bar) were measured by photolysis- chemiluminescence (Stamler, J.S., et al., Science, 276:2034-2037, 1997). Data shown are the average of 12 experiments ± SE. (D) Measurement of intraerythrocytic *S*-nitrosoHb and iron nitrosylHb formed by exposure of oxygenated RBCs (mean [Hb], 25 µM) to 0.3 µM NO. Isolation of Hb and measurements were as previously described (Stamler, J.S., et al., Science, 276:2034-2037, 1997). Data are the mean of 12 experiments ± SE.

### Example 5. EPR spectroscopy reveals the chemical dynamics of the iron-nitrosyl group in human hemoglobin, in response to oxygenation/deoxygenation

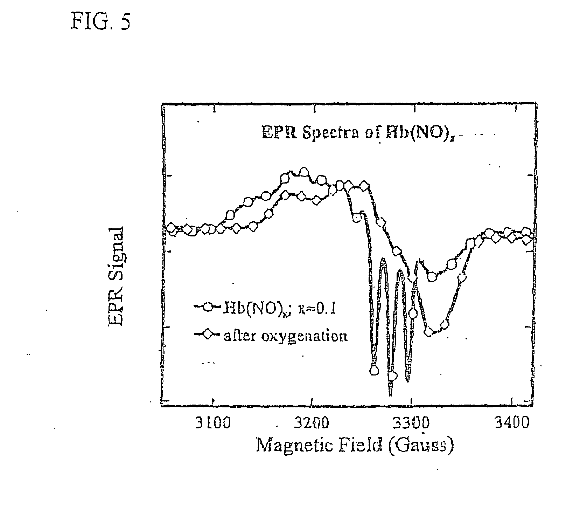

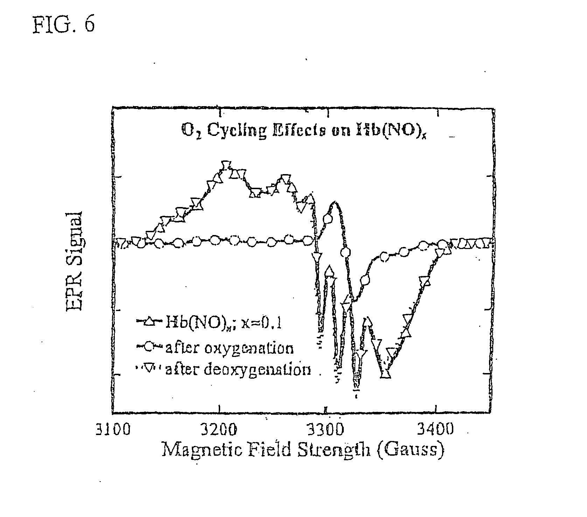

A predominantly α-T (nitrosyl) spectrum gains substantial P (and otherwise α-R) nitrosyl character on oxygenation (Figure 5). The iron-nitrosyl spectrum is reversibly eliminated upon oxygen cycling (Figure 6), in the presence of a redox mediator -- notably SNO is EPR silent and reforms nitrosyls with which it is in equilibrium upon deoxygenation. Results that unequivocally demonstrate the redistribution of NO-groups upon oxygenation can be seen in Figures 5 and 6. The EPR spectra in Figure 5 derive from a sample prepared by 5% NO saturation of deoxyHb. The initial spectrum, by decomposition, is seen to be largely α-T HbNO; upon oxygenation there is a decided change in the appearance of the spectrum, with the clear emergence of the β-subunit spectral component. This change seems to also be accompanied by a small loss in NO. To substantiate this, we performed EPR analyses of Fe-NO and chemiluminescence analyses of SNO-Hb on the same samples. The results presented in Figure 6 are more dramatic. This experiment is analogous to that of Figure 5, but nitrite, a redox mediator, is included in the medium. The initial spectrum shows a roughly equal mix of the three spectral components; upon oxygenation the entire Fe-NO spectrum disappears, indicating that all NO has come off the heme, and a small free-radical signal appears. After re-deoxygenation, the original spectrum is restored.

Conditions were, for Figure 5, 400 µl Hb (Apex) + 288 µl phosphate-EDTA, pH 7.4; phosphate = 85 mM; final heme concentration 520 µM (via UV-visible spectroscopy); 35 µM NO. Diamonds: deoxy sample was incubated 80 minutes. Circles: same sample was oxygenated.

Conditions were, for Figure 6, Hb (Apex) 650 µM final heme concentration (same up to 1 mM heme); nitrite:heme 1:4 (same for 1:1); 95 mM phosphate/EDTA. Incubation was for 1 minute as deoxy, followed by oxygenation, and re-deoxygenation.

EPR parameters: 9.274 GHz; 76 K (4 min sweep/0.25 sec time constant/10 mW microwave power/modulation amplitude 10G).

### Example 6. Distribution of NO in red blood cells

As the first step in analyzing the fate ofHb-derived NO *in situ,* we determined the disposition of NO transferred physiologically from hemes of Hb to cysβ93 in intact human erythrocytes (Gow, A.J., et al., Nature, 391:169-173, 1998; Gow, A.J., et al., Proc. Natl. Acad. Sci. USA, 96:9027-9032, 1999). RBCs held at <1% O₂ were exposed for 5 minutes to physiological amounts of NO (100 nM-1 mM; NO:heme ratios of 1:4000 to 1:100) followed by reoxygenation (21% O₂), and membrane and cytosolic fractions were prepared. Fractions were solubilized with Triton X-100 (TX-100), and the NO content of extracts was measured by photolysis/chemiluminescence (Gow, A.J., et al., Nature, 391:169-173, 1998; Gow, A.J., et al., Proc. Natl. Acad. Sci. USA, 96:9027-9032, 1999). At the lower NO:heme ratios, which produced intracellular NO concentrations matching those found *in vivo* (100-800 nM), recovery of NO was essentially complete, i.e. none was lost to nitrate (Figure 7A). About 15-20% of NO incorporated by RBCs was present as SNO; the remainder was ascribed largely to iron nitrosyl heme (Fe[II]NO) (Jia, L., et al., Nature, 380:221-226, 1996; Gow, A.J., et al., Nature, 391:169-173, 1998; Gow, A.J., et al., Proc. Natl. Acad. Sci. USA, 96:9027-9032, 1999; McMahon, T.J., et al., Meth. Enzymol., 301:99-114, 1999). Most iron nitrosyl Hb was recovered with the cytosolic fraction (Figure 7B). In contrast, SNO was associated predominantly with the membrane fraction (Figure 7C). These results demonstrate that in intact RBCs as with isolated reactants (Gow, A.J., et al., Nature, 391:169-173, 1998; Gow, A.J., et al., Proc. Natl. Acad. Sci. USA, 96:9027-9032, 1999), Hb will efficiently capture and preserve NO, and form SNO, under physiological conditions. Unexpectedly, however, the formation of SNO is compartmentalized within the RBC.

Hemoglobin binds to the cytoplasmic face of the RBC membrane both non-specifically and through specific protein-protein interactions (Rauenbuehler, P.B., et al., Biochim. Biophys. Acta, 692:361-370, 1982; Walder, J.A., et al., J. Biol. Chem., 259:10238-10246, 1984; Low, P.S., Biochim. Biophys. Acta, 864:145-167, 1986), and we found that the membrane fraction derived by osmotic lysis at physiological pH contained about 5% of total RBC Hb, which could not easily be removed under conditions that preserved SNO (not shown). To determine the disposition of Hb-derived and membrane-associated SNO. we examined the interaction of SNO-Hb (McMahon, T.J., et al., J. Biol. Chem., 275:16738-16745, 2000; McMahon, T.J., et al., Meth. Enzymol., 301:99-114, 1991) with inside-out vesicles (IOV) prepared by everting RBC membrane ghosts (Kondo, T., Meth. Enzymol., 171:217-225, 1989).

IOVs incubated with SNO-Hb and washed at pH 8 to remove bound Hb incorporated about 450 pmol NO/milligram of TX-100-extracted IOV protein (Figure 7D). Incorporated NO was present entirely in complex with thiol, i.e. as SNO. It is important to note that SNO was not detected in extracts of IOVs exposed to NO in the absence of Hb (not shown). To rule out the possibility that NO group transfer to the IOV was an artefactual consequence of detergent solubilization of residual membrane-bound SNO-Hb, we incubated IOVs with SNO-Hb immobilized on Sephadex beads. Following centrifugal separation, washes at pH 7 and solubilization in TX-100, extracts of IOVs were free of Hb as assessed by spectrophotometric detection of heme. SNO was present in those extracts at levels somewhat higher than in extracts derived from IOVs incubated with free SNO-Hb (suggesting greater loss of SNO from IOVs at pH 8 than at pH 7) (Figure 7D). In addition, incorporation of NO was inhibited substantially by prior, specific modification of exposed and reactive IOV thiols with the organic mercurial p-chloromercuriphenylsulfonic acid (PCMPS) (Figure 7D). Incorporation of NO groups was inhibited equally following mild digestion of IOVs with chymoptrypsin to remove protein domains external to the cytoplasmic membrane face (Figure 7D). Taken together, these results indicate that the NO group is transferred by SNO-Hb to cysteine sulfhydryls exposed at the inner surface of the RBC membrane.

### Example 7. AE1 accepts NO groups transferred from SNO-hemoglobin

The principal interaction of Hb with the RBC membrane is through specific, high-affinity binding to the N-terminal cytoplasmic domain of the chloride/bicarbonate anion exchange protein AE1 (band 3 protein) (Rauenbuehler, P.B., et al., Biochim. Biophys. Acta, 692:361-370, 1982; Walder, J.A., et al., J. Biol. Chem., 259:10238-10246, 1984; Low, P.S., Biochim. Biophys. Acta, 864:145-167, 1986). This domain contains two cysteine residues with reactive thiols that are removed by chymotrypsin (Rauenbuehler, P.B., et al., Biochim. Biophys. Acta, 692:361-370, 1982; Low, P.S., Biochim. Biophys. Acta, 864:145-167, 1986) and that are surrounded by amino acids that fit an *S*-nitrosylation motif (Stamler, J.S., et al., Neuron, 18:691-696, 1997). Further, AE1 can be oxidatively cross-linked to bound Hb (and SNO-Hb; our unpublished observations) through a disulfide bond involving the reactive β-globin thiol (Sayare, M., et al., J. Biol. Chem., 256:13152-13158, 1981). Therefore, transnitrosylation of a vicinal thiol within the cytoplasmic domain of AE1 is a strong candidate mechanism for transfer of the NO group from cysβ93 of SNO-Hb to the RBC membrane.

To assess this mechanism, we incubated IOVs with free or Sepharose-bound SNO-Hb and also treated RBCs with NO (NO:heme ratio of 1:250), prepared TX-100 or NP-40 extracts of IOVs and RBC membranes, immunoprecipitated AE1 with monoclonal antibodies, and measured NO in immunoprecipitates (IPs). With standardized quantities of antibody (AE1 in excess), IPs generated from extracts of either RBC membranes or IOVs incubated with free SNO-Hb contained about 60-80 pmol of NO, all of which was present as SNO (Figures 8A, 8C). Levels of SNO were substantially higher in IPs of AE1 from IOVs incubated with immobilized rather than free SNO-Hb (Figure 8B). SNO was detected at negligible levels in IPs generated with a non-specific mouse IgG (Figures 8A-8C). As an additional control for the specificity of association of SNO with immunoprecipitated AE1, we measured the SNO content of IPs generated with a monoclonal antibody to glycophorin, an abundant RBC membrane protein which also binds Hb ((Rauenbuehler, P.B., et al., Biochim. Biophys. Acta. 692:361-370, 1982) but which contains no cysteine. The SNO content of glycophorin IPs was minimal (Figure 8A).

Further evidence for a central role of AE1 in transfer of the NO moiety from SNO-Hb to the RBC membrane was provided by an analysis of the effects on transnitrosylation of prior treatment of RBCs with the disulfonic stilbene derivative, 4,4'-diisothiocyanatostilbene-2,2'-disulfonic acid (DIDS). Stilbene disulfonates have been employed extensively as specific inhibitors of electroneutral anion exchange and thus of AE1 function in RBCs (Falke, J.J., et al., Biochemistry, 25:7888-7894, 1986). DIDS acts on AE1 through covalent modification of identified lysine residues within the anion transporter (Falke, J.J., et al., Biochemistry, 25:7888-7894, 1986; Okubo, K., et al., J. Biol. Chem., 269:1918-1926, 1994), and that modification induces changes in the conformation of AE1 which can be detected not only in the C-terminal bilayer-spanning domain but also in the N-terminal cytoplasmic domain (Salhany, J.M., et al., Biochemistry, 19:1447-1454, 1980; Hsu, L., et al., Archiv. Biochem. Biophys., 227:31-38, 1983; Macara, I.G., et al., J. Biol. Chem., 258:1785-1792, 1983).

Incubation of intact RBCs with DIDS before exposure to NO (NO:heme ratio of 1:250) did not reduce the net formation of NO-liganded Hb, but greatly decreased the amount of SNO detected in the membrane fraction (Figure 8D). Further, SNO content was reduced substantially in IPs of AE1 from membrane extracts of RBCs treated with DIDS before exposure to NO (Figure 8E). Transfer of NO groups from SNO-Hb to IOVs prepared from DIDS-treated RBCs was reduced significantly, to a level comparable to that seen after modification of IOV thiols with PCMPS or following chymotryptic digestion of IOV proteins (Figure 8E). Evidence that DIDS did not affect SNO-Hb binding *per se,* but rather specifically altered the interaction with AE1 required for transnitrosylation, was provided by the finding that SNO-Hb bound with equal affinity to IOVs derived from native or DIDS-treated RBCs (Figure 8F). In addition, we determined that DIDS did not modify sulfhydryls directly, by comparing alkylation with ¹⁴C-iodoacetamide of proteins from DIDS-modified and unmodified IOVs (Figure 8G). Thus, inhibition of transnitrosylation by DIDS supports the conclusion that AE1 is the principal acceptor of NO groups transferred from SNO-Hb to the RBC membrane.

### Example 8. AE1 blocks vasorelaxant activity at low pO₂ of red blood cells previously exposed to NO

If RBCs in the systemic circulation transform endothelial-derived NO into SNO and then export physiological amounts of this activity to dilate blood vessels in a process regulated by pO₂ (Jia, L., et al., Nature, 380:221-226, 1996; Stamler, J.S., et al., Science, 276:2034-2037, 1997; McMahon, T.J., et al., J. Biol. Chem., 275:16738-16745, 2000), and if that process is dependent at least in part upon the transnitrosylative mechanism described by our results, then RBCs exposed *in vitro* to low level (nanomolar) NO should be capable of promoting oxygen-regulated relaxation of arterial smooth muscle, and release of this vasodilatory activity should be inhibited by DIDS treatment (that interferes with NO group transfer from SNO-Hb to the RBC membrane). We tested these predictions employing a standard bioassay based on measuring the tension developed by ring segments of rabbit thoracic aorta in medium at specified pO₂ (Jia, L., et al., Nature, 380:221-226, 1996; Stamler, J.S., et al., Science, 276:2034-2037, 1997).

We first determined that *S*-nitrosylated IOVs (at nanomolar concentrations) can relax aortic rings and thus that physiological quantitites of membrane-associated SNO can convey bioactivity (Figure 9A). In bioassay medium at 95% O₂, addition of RBCs (bath hematocrit of 0.4%; 80 mM heme) previously exposed to 400 nM NO (NO:heme ratio of 1:250) elicited contraction of aortic rings, which was of similar magnitude to that produced by RBCs treated with DIDS before exposure to NO and by control RBCs (which had undergone deoxygenation/reoxygenation as for NO treatment) (Figure 9B). This observation is consistent with previous studies demonstrating that free oxyHb and SNO-Hb increase aortic smooth muscle tone at high pO₂ (Jia, L., et al., Nature, 380:221-226, 1996; Stamler, J.S., et al., Science, 276:2034-2037, 1997; McMahon, T.J., et al., J. Biol. Chem., 275:16738-16745, 2000; McMahon, T.J., et al., Meth. Enzymol., 301:99-114, 1999), which can be ascribed to scavenging by R-structured Hb of NO produced by basal activity of endothelial nitric oxide synthase (McMahon, T.J., et al., J Biol. Chem., 275:16738-16745, 2000; McMahon, T.J., et al., Meth. Enzymol., 301:99-114, 1999). In marked contrast, aortic tone in medium at <1% O₂ was relaxed significantly by addition of RBCs previously exposed to NO (60 nM final SNO concentration, as for *S-*nitrosylated IOVs) (Figures 9A and 9B). Further, RBCs treated with DIDS before exposure to NO produced substantially less relaxation, to the extent that the average response did not differ significantly from that evoked by control RBCs at <1% O₂ (Figures 9A and 9B). It is important to recognize that NO was added to RBCs many minutes before they were employed, and thus that RBCs preserved NO bioactivity to release it on demand. These results demonstrate that transition from high to low pO₂ evokes the export of vasodilatory NO bioactivity from RBCs (containing physiological amounts of NO), and show that release is inhibited by disrupting the transnitrosylative transfer of NO groups from SNO-Hb to cysteine thiols within the cytoplasmic domain of AE1.

In addition, it is notable that control RBCs had little effect on aortic tone at <1% O₂ (Figure 9B). This observation contrasts markedly with the increase in tone elicited by free Hb, which is not reduced at low pO₂ (Stamler, J.S., et al., Science, 276:2034-2037, 1997; McMahon, T.J., et al., Meth., Enzymol., 301:99-114, 1999), and suggests that net import of extracellular (endothelial-derived) NO to cytosolic Hb is inhibited under those conditions where conjoint delivery of O₂ and NO bioactivity is called for.

While this invention has been particularly shown and described with references to preferred embodiments thereof, it will be understood by those skilled in the art that various changes in form and details may be made therein without departing from the scope of the invention encompassed by the appended claims.

## Claims

1. A method for determining the predominant physiological effect of a composition comprising hemoglobin when used in a method of therapy comprising the steps of:
a) obtaining EPR or UV spectra of iron-nitrosyl hemoglobin derivates formed by incubation of NO as a limiting reagent with hemoglobin at various degrees of oxygen saturation;
b) determining from the results in a) whether the composition shows non-cooperativity or cooperativity in binding of NO to the hemoglobin; and
c) if the composition shows cooperativity, then assaying the composition at an oxygen saturation at which the hemoglobin is approximately 99% oxyhemoglobin and 1% deoxyhemoglobin, under limiting NO concentration, to determine whether *S*-nitroso-hemoglobin or iron nitrosyl-hemoglobin is greater;
wherein, if the composition shows non-cooperativity, then the predominant physiological effect of the composition is elimination of NO; if the composition shows cooperativity and if *S*-nitroso-hemoglobin is greater, then the predominant physiological effect of the composition is delivering NO; and if the composition shows cooperativity and if iron nitrosyl-hemoglobin is greater, then the predominant physiological effect of the composition is trapping of NO.

2. A method for determining the predominant physiological effect of a composition comprising hemoglobin when used in a method of therapy, comprising the steps of:
a) obtaining EPR or UV spectra of iron-nitrosyl hemoglobin derivatives formed by incubation of NO as a limiting reagent with hemoglobin at various degrees of oxygen saturation;
b) determining from the results in a) whether the composition shows non-cooperativity or cooperativity in binding of NO to the hemoglobin; and
c) if the composition shows cooperativity, then assaying the composition at an oxygen saturation at which the hemoglobin is approximately 99% oxyhemoglobin and 1% deoxyhemoglobin, under limiting NO concentration, to determine whether *S*-nitroso-hemoglobin or iron nitrosyl-hemoglobin is greater;
wherein, if the composition shows non-cooperativity, then the predominant physiological effect of the composition is vasoconstriction; if the composition shows cooperativity and if the most prevalent species of NO-modified hemoglobin is *S*-nitroso-hemoglobin, then the predominant physiological effect of the composition is vasodilation; and if the composition shows cooperativity and if iron nitrosyl-hemoglobin is greater, then the predominant physiological effect of the composition is vasoconstriction.

3. A method for determining the predominant physiological effect of a blood sample from a patient, comprising the steps of:
a) obtaining EPR or UV spectra of iron-nitrosyl hemoglobin derivatives formed by incubation of NO as a limiting reagent with hemoglobin at various degrees of oxygen saturation;
b) determining from the results in a) whether the sample shows non-cooperativity or cooperativity in binding of NO to the hemoglobin; and
c) if the sample shows cooperativity, then assaying the sample at an oxygen saturation at which the hemoglobin is approximately 99% oxyhemoglobin and 1% deoxyhemoglobin, under limiting NO concentration, to determine whether *S*-nitroso-hemoglobin or iron nitrosyl-hemoglobin is greater;
wherein, if the sample shows cooperativity, and the most prevalent species of NO-modified hemoglobin is *S*-nitroso-hemoglobin, then concluding that the predominant physiological effect of the sample is vasodilation.

## Patentansprüche

1. Verfahren zur Bestimmung der vorherrschenden physiologischen Wirkung einer Zusammensetzung, die Hämoglobin aufweist, wenn sie in einem Therapieverfahren verwendet wird, folgende Schritte aufweisend:
a) Erhalten von EPR- oder UV-Spektren von Eisen-Nitrosylhämoglobin-Derivaten, die durch Inkubation von NO als limitierendes Reagenz mit Hämoglobin bei verschiedenen Sauerstoff-Sättigungsgraden gebildet wurden;
b) Bestimmen aus den Ergebnissen in a), ob die Zusammensetzung Nicht-Kooperativität oder Kooperativität bei der Bindung von NO an das Hämoglobin zeigt; und
c) wenn die Zusammensetzung Kooperativität zeigt, dann Untersuchen der Zusammensetzung bei einer Sauerstoff-Sättigung, bei der das Hämoglobin zu näherungsweise 99% Oxyhämoglobin und zu 1 % Desoxyhämoglobin ist, unter limitierender NO-Konzentration, um zu bestimmen, ob mehr S-Nitrosohämoglobin oder Eisen-Nitrosylhämoglobin vorliegt;
wobei, wenn die Zusammensetzung Nicht-Kooperativität zeigt, die vorherrschende physiologische Wirkung der Zusammensetzung dann Beseitigung von NO ist; wenn die Zusammengesetzt Kooperativität zeigt und wenn mehr S-Nitrosohämoglobin vorliegt, die vorherrschende physiologische Wirkung der Zusammensetzung dann Abgabe von NO ist; und wenn die Zusammensetzung Kooperativität zeigt und wenn mehr Eisen-Nitrosylhämoglobin vorliegt, die vorherrschende physiologische Wirkung der Zusammensetzung dann Abfangen von NO ist.

2. Verfahren zur Bestimmung der vorherrschenden physiologischen Wirkung einer Zusammensetzung, die Hämoglobin aufweist, wenn sie in einem Therapieverfahren verwendet wird, folgende Schritte aufweisend:
a) Erhalten von EPR- oder UV-Spektren von Eisen-Nitrosylhämoglobin-Derivaten, die durch Inkubation von NO als limitierendes Reagenz mit Hämoglobin bei verschiedenen Sauerstoff-Sättigungsgraden gebildet wurden;
b) Bestimmen aus den Ergebnissen in a), ob die Zusammensetzung Nicht-Kooperativität oder Kooperativität bei der Bindung von NO an das Hämoglobin zeigt; und
c) wenn die Zusammensetzung Kooperativität zeigt, dann Untersuchen der Zusammensetzung bei einer Sauerstoff-Sättigung, bei der das Hämoglobin zu näherungsweise 99% Oxyhämoglobin und zu 1% Desoxyhämoglobin ist, unter limitierender NO-Konzentration, um zu bestimmen, ob mehr S-Nitrosohämoglobin oder Eisen-Nitrosylhämoglobin vorliegt;
wobei, wenn die Zusammensetzung Nicht-Kooperativität zeigt, die vorherrschende physiologische Wirkung der Zusammensetzung dann Vasokonstriktion ist; wenn die Zusammengesetzt Kooperativität zeigt und wenn die am meisten vorherrschende Spezies von NO-modifiziertem Hämoglobin S-Nitrosohämoglobin ist, die vorherrschende physiologische Wirkung der Zusammensetzung dann Vasodilatation ist; und wenn die Zusammensetzung Kooperativität zeigt und wenn mehr Eisen-Nitrosylhämoglobin vorliegt, die vorherrschende physiologische Wirkung der Zusammensetzung dann Vasokonstriktion ist.

3. Verfahren zur Bestimmung der vorherrschenden physiologischen Wirkung einer Blutprobe von einem Patienten, folgende Schritte aufweisend:
a) Erhalten von EPR- oder UV-Spektren von Eisen-Nitrosylhämoglobin-Derivaten, die durch Inkubation von NO als limitierendes Reagenz mit Hämoglobin bei verschiedenen Sauerstoff-Sättigungsgraden gebildet wurden;
b) Bestimmen aus den Ergebnissen in a), ob die Probe Nicht-Kooperativität oder Kooperativität bei der Bindung von NO an das Hämoglobin zeigt; und
c) wenn die Probe Kooperativität zeigt, dann Untersuchen der Probe bei einer Sauerstoff-Sättigung, bei der das Hämoglobin zu näherungsweise 99% Oxyhämoglobin und zu 1% Desoxyhämoglobin ist, unter limitierender NO-Konzentration, um zu bestimmen, ob mehr S-Nitrosohämoglobin oder Eisen-Nitrosylhämoglobin vorliegt;
wobei, wenn die Probe Kooperativität zeigt und die am meisten vorherrschende Spezies des NO-modifizierten Hämoglobins S-Nitrosohämoglobin ist, dann Schlussfolgern, dass die vorherrschende physiologische Wirkung der Probe Vasodilatation ist.

## Revendications

1. Procédé pour déterminer l'effet physiologique prédominant d'une composition comprenant de l'hémoglobine quand elle est utilisée dans une méthode de thérapie, comprenant les étapes :
a) d'obtention des spectres EPR ou UV de dérivés de fer-nitrosyl hémoglobine formés par incubation de NO, servant de réactif limitant, avec de l'hémoglobine, à divers degrés de saturation en oxygène ;
b) de détermination, à partir des résultats obtenus en a), si la composition présente une non-coopérativité ou une coopérativité dans la liaison de NO à l'hémoglobine ; et
c) si la composition présente une coopérativité, de test de la composition à une saturation en oxygène à laquelle l'hémoglobine est constituée à environ 99 % d'oxyhémoglobine et 1 % de désoxyhémoglobine, à une concentration limitante de NO, pour déterminer s'il y a davantage de S-nitroso-hémoglobine ou de fer-nitrosyl hémoglobine ;
dans lequel, si la composition présente une non-coopérativité, alors l'effet physiologique prédominant de la composition est l'élimination de NO ; si la composition présente une coopérativité et s'il y a davantage de S-nitroso-hémoglobine, alors l'effet physiologique prédominant de la composition est la libération de NO ; et si la composition présente une coopérativité et s'il y a davantage de fer-nitrosyl hémoglobine, alors l'effet physiologique prédominant de la composition est le piégeage de NO.

2. Procédé pour déterminer l'effet physiologique prédominant d'une composition comprenant de l'hémoglobine quand elle est utilisée dans une méthode de thérapie, comprenant les étapes :
a) d'obtention des spectres EPR ou UV de dérivés de fer-nitrosyl hémoglobine formés par incubation de NO, servant de réactif limitant, avec de l'hémoglobine, à divers degrés de saturation en oxygène ;
b) de détermination, à partir des résultats obtenus en a), si la composition présente une non-coopérativité ou une coopérativité dans la liaison de NO à l'hémoglobine ; et
c) si la composition présente une coopérativité, de test de la composition à une saturation en oxygène à laquelle l'hémoglobine est constituée à environ 99 % d'oxyhémoglobine et 1 % de désoxyhémoglobine, à une concentration limitante de NO, pour déterminer s'il y a davantage de S-nitroso-hémoglobine ou de fer-nitrosyl hémoglobine ;
dans lequel, si la composition présente une non-coopérativité, alors l'effet physiologique prédominant de la composition est la vasoconstriction ; si la composition présente une coopérativité et si l'espèce la plus répandue d'hémoglobine NO-modifiée est la S-nitroso-hémoglobine, alors l'effet physiologique prédominant de la composition est la vasodilatation ; et si la composition présente une coopérativité et s'il y a davantage de fer-nitrosyl hémoglobine, alors l'effet physiologique prédominant de la composition est la vasoconstriction.

3. Procédé pour déterminer l'effet physiologique prédominant d'un échantillon de sang provenant d'un patient, comprenant les étapes :
a) d'obtention des spectres EPR ou UV de dérivés de fer-nitrosyl hémoglobine formés par incubation de NO, servant de réactif limitant, avec de l'hémoglobine, à divers degrés de saturation en oxygène ;
b) de détermination, à partir des résultats obtenus en a), si l'échantillon présente une non-coopérativité ou une coopérativité dans la liaison de NO à l'hémoglobine ; et
c) si l'échantillon présente une coopérativité, de test de l'échantillon à une saturation en oxygène à laquelle l'hémoglobine est constituée à environ 99 % d'oxyhémoglobine et 1 % de désoxyhémoglobine, à une concentration limitante de NO, pour déterminer s'il y a davantage de S-nitroso-hémoglobine ou de fer-nitrosyl hémoglobine ;
dans lequel, si l'échantillon présente une coopérativité, et si l'espèce la plus répandue d'hémoglobine NO-modifiée est la S-nitroso-hémoglobine, il en est alors conclu que l'effet physiologique prédominant de l'échantillon est la vasodilatation.
